# EUROPÄISCHE PATENTSCHRIFT

(11) **EP 2 848 262 B1**
(45) Veröffentlichungstag und Bekanntmachung des Hinweises auf die Patenterteilung: **04.11.2020**
(21) Anmeldenummer: 13184146.2
(22) Anmeldetag: 12.09.2013
(51) Int. Cl.: A61K 47/69, A61K 47/54, A61K 49/00, A61K 31/713, A61P 1/16, A61P 13/12, A61P 35/00, A61P 43/00

(54) **Zellspezifisches Targeting durch Nanostrukturierte Trägersysteme**
Cell-specific targeting by nanostructured carrier systems
Ciblage spécifique à des cellules par des systèmes porteurs nanostructurés

(43) Veröffentlichungstag der Anmeldung: 18.03.2015
(73) Patentinhaber: SmartDyeLivery GmbH, 07743 Jena (DE)
(72) Erfinder: Prof. Dr. Bauer, Michael, 07743 Jena (DE); Prof. Dr. Schubert, Ulrich, 07743 Jena (DE); Dr. Gottschaldt, Michael, 07749 Jena (DE); Dr. Schallon, Anja, 95367 Trebgast (DE); Pietsch, Christian, 07743 Jena (DE); Dr. Gonnert, Falk, 07745 Jena (DE); Dr. Recknagel, Peter, 98529 Albrechts (DE); Press, Adrian, 07747 Jena (DE)
(74) Vertreter: Kehl, Ascherl, Liebhoff & Ettmayr Patentanwälte Partnerschaft mbB

(56) Entgegenhaltungen:
- WO-A1-2013/051732
- PARUL RUNGTA ET AL: "Selective Imaging and Killing of Cancer Cells with Protein-Activated Near-Infrared Fluorescing Nanoparticles", MACROMOLECULAR BIOSCIENCE, Bd. 11, Nr. 7, 8. April 2011 (2011-04-08), Seiten 927-937, XP055056114, ISSN: 1616-5187, DOI: 10.1002/mabi.201100043
- YOUNG-WOOCK NOH ET AL: "Near-Infrared Emitting Polymer Nanogels for Efficient Sentinel Lymph Node Mapping", ACS NANO, Bd. 6, Nr. 9, 25. September 2012 (2012-09-25), Seiten 7820-7831, XP055097717, ISSN: 1936-0851, DOI: 10.1021/nn301949y
- YEH CHEN-SHENG ET AL: "Tumor targeting and MR imaging with lipophilic cyanine-mediated near-infrared responsive porous Gd silicate nanoparticles", BIOMATERIALS, Bd. 34, Nr. 22, 29. April 2013 (2013-04-29), Seiten 5677-5688, XP028535344, ISSN: 0142-9612, DOI: 10.1016/J.BIOMATERIALS.2013.04.020
- N. JIANG ET AL: "Targeted Gene Silencing of TLR4 Using Liposomal Nanoparticles for Preventing Liver Ischemia Reperfusion Injury", AMERICAN JOURNAL OF TRANSPLANTATION, Bd. 11, Nr. 9, 27. Juli 2011 (2011-07-27), Seiten 1835-1844, XP055097701, ISSN: 1600-6135, DOI: 10.1111/j.1600-6143.2011.03660.x
- CHENG JIN ET AL: "Improved Therapeutic Effect of DOX-PLGA-PEG Micelles Decorated with Bivalent Fragment HAb18 F(ab') 2 for Hepatocellular Carcinoma", BIOMACROMOLECULES, Bd. 11, Nr. 9, 13. September 2010 (2010-09-13), Seiten 2422-2431, XP055097742, ISSN: 1525-7797, DOI: 10.1021/bm1005992
- ELVIN BLANCO ET AL: "Principles of nanoparticle design for overcoming biological barriers to drug delivery", NATURE BIOTECHNOLOGY, vol. 33, no. 9, 1 September 2015 (2015-09-01), pages 941-951, XP055636478, us ISSN: 1087-0156, DOI: 10.1038/nbt.3330
- KAI XIAO ET AL: "The effect of surface charge onbiodistribution of PEG-oligocholic acid based micellar nanoparticles", BIOMATERIALS, ELSEVIER, AMSTERDAM, NL, vol. 32, no. 13, 6 January 2011 (2011-01-06), pages 3435-3446, XP028366487, ISSN: 0142-9612, DOI: 10.1016/J.BIOMATERIALS.2011.01.021 [retrieved on 2011-01-12]

## Beschreibung

Die vorliegende Erfindung betrifft nanostruktuierte Trägersysteme gemäß den Ansprüchen zur Verwendung für die Behandlung von Erkrankungen der Leber und/oder Niere, die ein oder mehrere Polymere und/oder Lipide sowie einen oder mehrere Polymethinfarbstoffe als Targetingeinheiten zum zielgerichteten Transport des nanostrukturieren Trägersystems in ein Zielgewebe umfassen. Die Erfindung betrifft weiterhin den zellspezifischen Transport eines oder mehrerer pharmazeutischer Wirkstoffe in ein spezifisches Zielgewebe (zellspezifisches Targeting) und die Verwendung der erfindungsgemäßen nanostrukturierten Trägersystem zur Prophylaxe und/oder Therapie von Erkrankungen der Leber und/oder Niere.

Im Stand der Technik ist die Verwendung von Nanopartikeln mit gekoppelten Farbstoffen in der klinischen Diagnostik bekannt, beispielsweise zur Detektion von Organfunktionen oder Proteinexpressionen in der Diagnose pathogener Zustände, oder für Proteomanalysen. Hierbei werden die gekoppelten Farbstoffe, in der Regel Fluoreszenzfarbstoffe, wie Cyanine, als Marker verwendet, deren Fluoreszenz- und Absorptionseigenschaften gemessen wird.

WO20121013247A1 beschreibt die Verwendung von Polymethinfluoreszenzfarbstoffen zur Bestimmung einer Organfunktion, insbesondere der Funktion von Leber oder Niere. Hierbei wird der Farbstoff als Marker in einem Gewebe oder einer Körperflüssigkeit, wie Blut oder Urin, verwendet und radiativ angeregt, es wird sodann die Fluoreszenzemission des Farbstoffs detektiert, die Daten erfasst und ausgewertet, um die untersuchte Organfunktion zu bestimmen.

In WO2010/116209A1 wird beschrieben, wie anhand von Fluoreszenzfarbstoffen und Fluoreszensspektrographie ein klinischer Zustand detektiert werden kann, der auf einer abnormalen Selectinsekretion basiert.

Rungta, P. et al., Selective Imaging and killing of cancer cells with protein-activated near-infrared fluorescing nanoparticles. Macromol Biosci, 2011. 11: S. 927-37 beschreibt Nanopartikel umfassend Indocyaningrün zur Darstellung und Behandlung von Leberzellkarzinom.

WO2013/051732A1 beschreibt Liposomen umfassend NIR-Farbstoffe zur Behandlung von Tumoren.

Im Stand der Technik ist weiterhin die Verwendung von Nanopartikeln bekannt, welche Wirkstoffe zielgerichtet in ein bestimmtes Gewebe transportieren (Sheridan, C., Proof of concept for next-generation nanoparticle drugs in humans. Nat Biotechnol, 2012. 30(6): S. 471-3; Gratton, S.E. et al., The effect of particle design on cellular intemalization pathways. Proc Natl Acad Sci U S A, 2008. 105(33): S. 11613-8; Jiang, N. et al., Targeted Gene Silencing of TLR4 using liposomal nanoparticles for preventing liver ischemia reperfusion injury. American Journal of Transplantation, 2011. 11: S. 1835-44).

Unter einem zielgerichteten oder zellspezifischen Transport eines Wirkstoffs, auch "drug targeting" oder "targeted drug delivery", versteht man die zielgerichtete und selektive Anreicherung und Freisetzung eines Wirkstoffs an einem gewünschten Wirkort, wobei die Effektivität in der Wirkung des Wirkstoffs erhöht und die systemischen Nebenwirkungen für das umliegende Gewebe verringert werden soll. Transportierte Wirkstoffe sind häufig Antikörper, Peptide oder kleine Moleküle, wie Oligonukleotide oder Nukleinsäuren.

Die im Stand der Technik bekannten Wirkstoff-transportierenden Nanopartikel werden in der Tumortherapie verwendet und funktionieren nach folgenden Mechanismen: Entweder wird der Nanopoartikel mit einer Hüllschicht oder einem Antikörper versehenen. Wird der Nanopartikel mit einer wässrigen Hüllschicht überzogen ist er für das Immunsystem unkenntlich gemacht. Wird dieser Nanopartikel injiziert und vom Immunsystem nicht angegriffen, diffundiert er durch die im Tumor "undichten" fernestierten Blutgefäße, die im Vergleich zu normalen Blutgefäßen deutlich größere Öffnungen (Fernestierungen) aufweisen, und wird durch die umliegenden Zellen, die ebenfalls im Vergleich zu gesunden Zellen eine erhöhte Durchlässigkeit aufweisen, über Endozytose aufgenommen. Nachteil ist, dass nicht nur die gewünschten Zellen den Nanopartikel aufnehmen, sondern auch andere (gesunde) Zellen, zu denen der Nanopartikel über die Blutgefäße unspezifisch transportiert wird. Dies kann schwere Nebenwirkungen hervorrufen. Weiterer Nachteil ist, dass dieser Transport auf Tumorgewebe beschränkt ist, d.h., ein Transport in andere Gewebe, wie beispielsweise Leber oder Niere, nicht erfolgen kann. Dieser Transport erfolgt passiv und die Aufnahme ist unspezifisch bzw. nicht selektiv. Bei dem zweiten Verfahren wird der Nanopartikel nach seiner Herstellung mit Antikörpern auf seiner Oberfläche versehen. Ziel dieser Konstrukte sind Zellen mit Antigenen, an die diese Antikörper binden. Auch dieser Transportmechanismus ist passiv und nicht selektiv.

Diese vorstehend beschriebenen Vorgänge der passiven Anreicherung von Nanopartikeln, Liposomen oder Makromolekülen wird als EPR-Effekt ("enhanced permeability and retention"; erhöhte Permeabilität und Retention") bezeichnet und ist ein passives "drug targeting". Wie erwähnt sind die Nachteile, dass diese Vorgänge des Transports nicht aktiv und nicht selektiv erfolgen.

Nirgendwo im Stand der Technik ist ein aktives und selektives Transport- bzw. Trägersystem beschrieben, dessen aktiver Transport über spezielle Targetingeinheiten selektiv in ein spezifisches Zielgewebe erfolgt und mit dem gleichzeitig (pharmazeutische) Wirkstoffe in das Zielgewebe transportiert werden können ("drug targeting "; zellspezifisches Targeting) und die Akkumulation des Trägersystems und ggf. des (pharmazeutischen) Wirkstoffs in dem Zielgewebe über die Targetingeinheit nicht nur bewirkt, sondern auch nachverfolgt und überprüft werden kann.

Es besteht daher Bedarf, ein verbessertes Transport- bzw. Trägersystem bereitzustellen, das einen aktiven und selektiven Transport von Trägern und Wirkstoffen in ein Zielgewebe bewirkt. Es besteht weiterhin Bedarf, ein solches Transport- bzw. Trägersystem für den Transport pharmazeutischer Wirkstoffe in der Behandlung von Erkrankungen einzusetzen.

Mit der vorliegenden Erfindung wird ein solches Transportsystem bereitgestellt. Die vorliegende Erfindung betrifft ein einzigartiges, vielfach kombinierbares, theragnostisches System um verschiedene pharmazeutische Wirkstoffe (beispielsweise hydrophile, lipophile, hydrophobe, amphiphile, anionische und kationische Substanzen) aktiv und selektiv in ein Zielgewebe zu transportieren (zielgerichteter oder zellspezifischer Wirkstofftransport oder "drug targeting").

Die vorliegende Erfindung betrifft in ihrem ersten Gegenstand ein nanostrukturiertes Trägersystem gemäß den Ansprüchen zur Verwendung für die Behandlung von Erkrankungen der Leber und/oder Niere, das mindestens ein Polymer und/oder mindestens ein Lipid und mindestens einen Polymethinfarbstoff umfasst, wobei der mindestens eine Polymethinfarbstoff als Targetingeinheit den zielgerichteten Transport des nanostrukturieren Trägersystems in ein Zielgewebe bewirkt.

Sofern das erfindungsgemäße nanostruktierte Trägersystem Polymere umfasst, wird es hierin als "Nanopartikel" bezeichnet, sofern es Lipide umfasst, wird es hierin als "Liposom" bezeichnet. Sofern das erfindungsgemäße nanostruktierte Trägersystem sowohl Polymere als auch Lipide umfasst, wird es hierin als "Nanopartikel" oder als "Liposom" bezeichnet. Dementsprechend werden erfindungsgemäß die Begriffe, "Nanopartikel" und "Liposom" synonym verwendet und betreffen ebenfalls ein nanostruktuiertes Trägersystem, das sowohl Polymere als auch Lipide umfasst.

Nanopartikel sind Strukturen, die kleiner als 1 µm sind und aus mehreren Molekülen aufgebaut sein können. Sie zeichnen sich allgemein durch ein höheres Oberflächen zu Volumen Verhältnis aus und bieten damit eine höhere chemische Reaktivität. Diese Nanopartikel können aus Polymeren bestehen, wobei sich diese durch die Wiederholung von bestimmten Einheiten (Monomeren) auszeichnen. Die Polymere werden durch die chemische Reaktion dieser Monomere kovalent miteinander verbunden (Polymerisation). Besitzen diese Polymere zum Teil hydrophobe Eigenschaften, können sie in wässriger Umgebung nanoskalige Strukturen (z.B. Nanopartikel, Mizellen, Vesikel) ausbilden. Durch ihre hydrophoben Eigenschaften können auch Lipide dazu verwendet werden Nanopartikel zu bilden (Mizellen, Liposome).

Eine bevorzugte Ausführungsform der vorliegenden Erfindung betrifft ein nanostrukturieres Trägersystem, wobei das mindestens eine Polymer ausgewählt ist aus der Gruppe bestehend aus Polyestern, Poly(meth)acrylaten, Polystyrol Derivaten, Polyamiden, Polyurethanen, Polyacrylnitrilen, Polytetrafluorethylenen, Siliconen, Polyethylenglycolen, Polyethylenoxiden und Polyoxazolinen und deren Copolymere, vorzugsweise in verschiedenster Zusammensetzung, wie z. B. Statistisch, Gradient, Alternierend, Block, Pfropf oder Stern Copolymere oder das mindestens eine Lipid ausgewählt ist aus der Gruppe bestehend aus gesättigten und ungesättigten Fettsäuren, bevorzugt Cholesterin, Palmethylsäure, Phospolipide, Sphingolipide und Glycolipide. Vorzugsweise ist das erfindungsgemäße Polymer bzw. Lipid ein biokompatibles Polymer bzw. Lipid.

Besonders bevorzugt handelt es sich bei dem erfindungsgemäßen Polymer um ein hydrophobes, hydrophiles, amphiphiles, anionisches und/oder kationisches Polymer. Insbesondere bevorzugt ist das Polymer ausgewählt aus der Gruppe bestehend aus PLGA, PLA, PCL, PGA, PDMAEMA, PMMA, PMAA, PEI, PEtOx, PEG.

Als "Targetingeinheit" im Sinne der Erfindung werden Substanzen bezeichnet, die den Transport des nanostrukturierten Trägersystems erfindungsgemäß aktiv und selektiv in ein spezifisches Zielgewebe bewirken. Erfindungsgemäße Targetingeinheiten sind Polymethinfarbstoffe. Die Begriffe "Targetingeinheit" und "Polymethinfarbstoff" werden erfindungsgemäß synonym verwendet.

Der Polymethinfarbstoff im Sinne der Erfindung ist ein symmetrisches oder unsymmetrisches Polymethin der allgemeinen Struktur I, II, III oder IV: 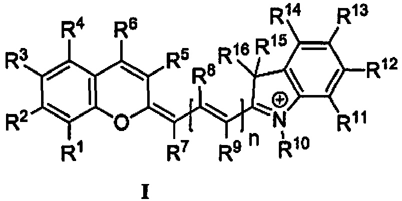 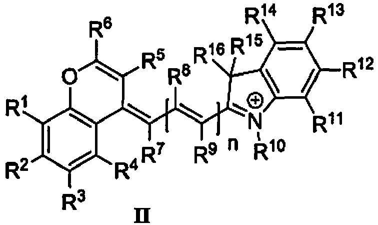 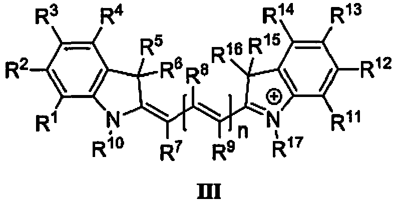 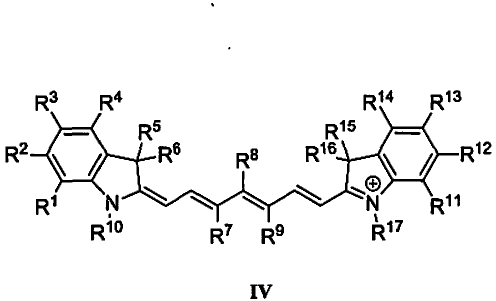 wobei
a. n für die Zahlenwerte 0, 1, 2, 3, 4, 5, 6, 7, 8, 9, oder 10 steht,
b. R¹- R¹⁷ gleich oder unterschiedlich sind und Wasserstoff, ein oder mehrere Alkyl-, tert-Alkyl, Cycloalkyl- (die Reste "Alkyl" und "Cycloalkyl" beinhalten auch Olefinische Strukturen) oder Aryl-, Carboxyaryl-, Dicarboxyaryl-, Heteroaryl- oder heterocycloaliphatische Reste, Alkyloxy-, Alkylmercapto, Aryloxy-, Arylmercapto-, Heteroaryloxy-, Heteroarylmercapto-, eine Hydroxy-, Nitro- oder Cyanogruppe, eine alkylsubsituierte oder cyclische Aminfunktion sein können und/oder zwei ortho-ständige Reste, z.B. R³ und R⁴, R¹³ und R¹⁴ und/oder R¹ und R² und R¹¹ und R¹² und/oder R⁷ und R⁹ zusammen einen weiteren aromatischen, hetero-aromatischen, aliphatischen oder heteroaliphatischen Ring bilden können,
c. mindestens einer der Substituenten R¹- R¹⁷ trägt einen solubilisierenden bzw. ionisierbaren oder ionisierten Substituenten, wie SO₃⁻, (-SO₃H), PO₃², COOH, OH oder NR₃⁺, Cyclodextrine oder Zucker, der die hydrophilen Eigenschaften dieser Polymethinfarbstoffe bestimmt, wobei dieser Substituent auch über eine Spacergruppe an dem Polymethinfarbstoff angebunden sein kann, und
d. mindestens einer der Substituenten R¹- R¹⁷ trägt eine reaktive Gruppe (Linker), wie Isocyanate, Isothiocyanate, Hydrazine, Amine, Mono- und Dichlor- oder Mono und Dibromtriazine, Aziridine, Epoxide, Sulfonylhalogenide, Säurehalogenide, Carbonsäureanhydride, N-Hydroxysuccinimidester, Imido-ester, Carbonsäuren, Glyoxal, Aldehyd, Maleimid oder lodacetamid und Phosphoramidit Derivate oder Azide, Alkine oder Olefine, wobei dieser Substituent auch über eine Spacergruppe an dem Polymethinfabstoff angebunden sein kann,
e. die aromatische, heteroaromatische, aliphatische oder heteroaliphatische Spacergruppe aus Strukturelementen, wie -[(CH₂)ₐ-Y-(CH₂)_{b}]_{c}- oder -[(C₆H₄)ₐ-Y-(C₆H₄)_{b}]- besteht, wobei Y gleich oder unterschiedlich ist und CR₂-, O-, S-, SO₂, SO₂NH-, NR-, COO- oder CONR Funktionen beinhalten, wobei sie an einem der Substituenten R¹- R¹⁷ ist und a und b gleich oder unterschiedlich sind mit Zahlenwerten von 0-18 und mit Zahlenwerten für c von 0-18,
f. die Substituenten R⁸ und R⁹ bei entsprechenden n= 2, 3, 4 oder 5 ebenfalls 2-,3-,4- oder 5-fach vorhanden sein können und diese gleich oder unterschiedlich sein können.

Erfindungsgemäß werden die Begriffe "Targetingeinheit" und "Polymethinfarbstoff" synonym verwendet.

Die Targetingeinheiten (Polymethinfarbstoffe) werden über einen Linker an das Polymer konjugiert.

### allgemeine Struktur Polymer - Linker - Targetingeinheit:

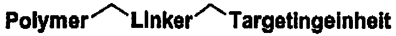

Die allgemeine Struktur eines erfindungsgemäßen Linkers ist wie folgt beschrieben: mindestens eine Struktureinheit (Polymer und/oder Targetingeinheit) trägt eine reaktive Gruppe (Linker), wie Isocyanate, Isothiocyanate, Hydrazine, Amine, Mono- und Dichlor- oder Mono und Dibromtriazine, Aziridine, Epoxide, Sulfonylhalogenide, Säurehalogenide, Carbonsäureanhydride, N-Hydroxysuccinimidester, Imido-ester, Carbonsäuren, Glyoxal, Aldehyd, Maleimid oder lodacetamid und Phosphoramidit Derivate oder Azide, Alkine oder Olefine, wobei dieser Substituent auch über eine Spacergruppe an dem Polymethinfabstoff und/oder das Polymer angebunden sein kann. Über diese reaktive Gruppe wird die Targetingeinheit mit dem Polymer (oder umgekehrt) über ein kovalent Bindung verknüpft.

Die chemische Bindungen zwischen Polymer bzw. Targetingeinheit und Linker können biostabil oder bioabbaubar gewählt werden. Es können eine oder mehrere verschiedene Targetingeinheiten an ein Polymer gebunden werden. Ebenfalls können mit verschiedenen Targetingeinheiten versehene Polymere in einem Nanopartikel gemischt werden. Dabei kann sich sowohl das Polymer als auch die Targetingeinheit als auch beides unterscheiden. Statt eines Polymers kann unter denselben Bedingungen wie vorstehend ausgeführt, anstelle eines Polymers auch ein Lipid, und entsprechend anstelle eines Nanopartikels auch ein Liposom, verwendet werden.

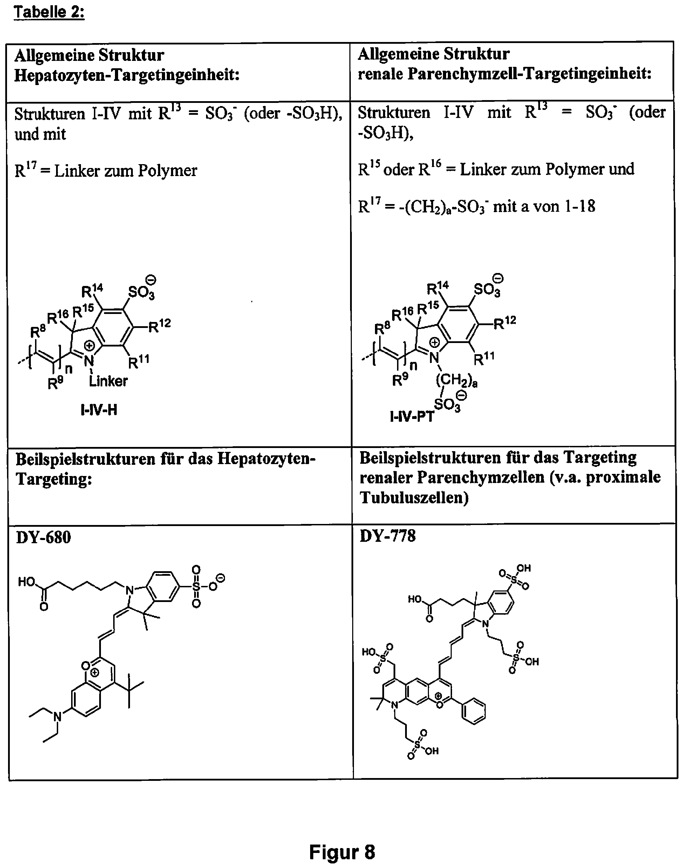

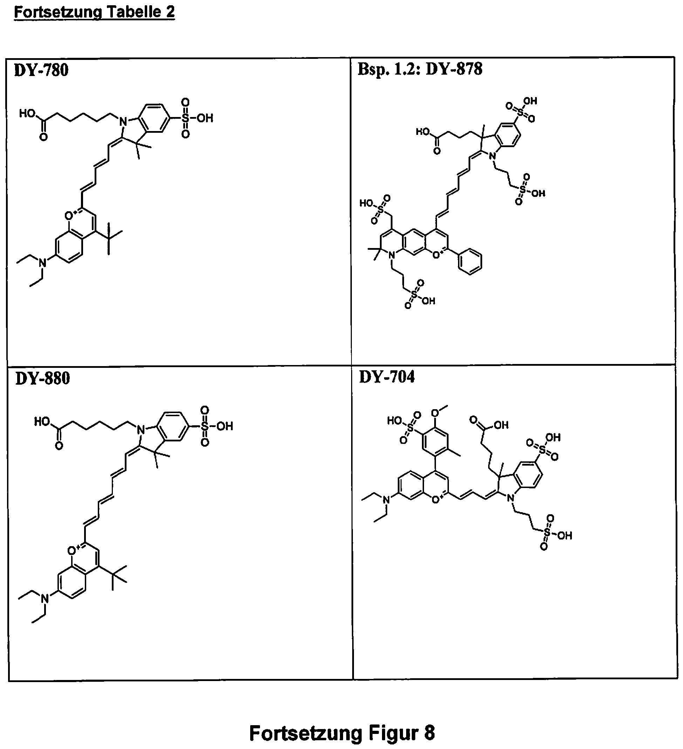

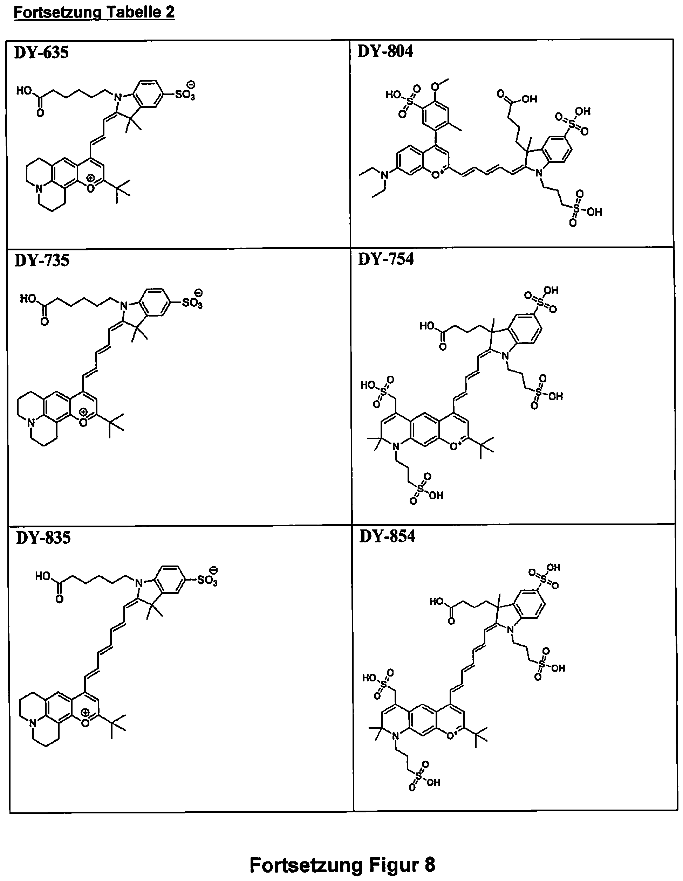

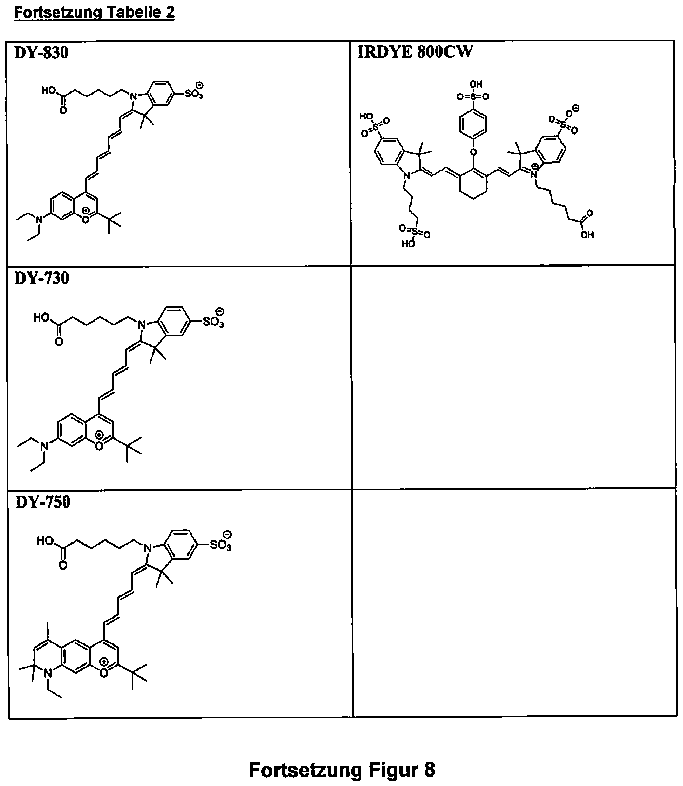

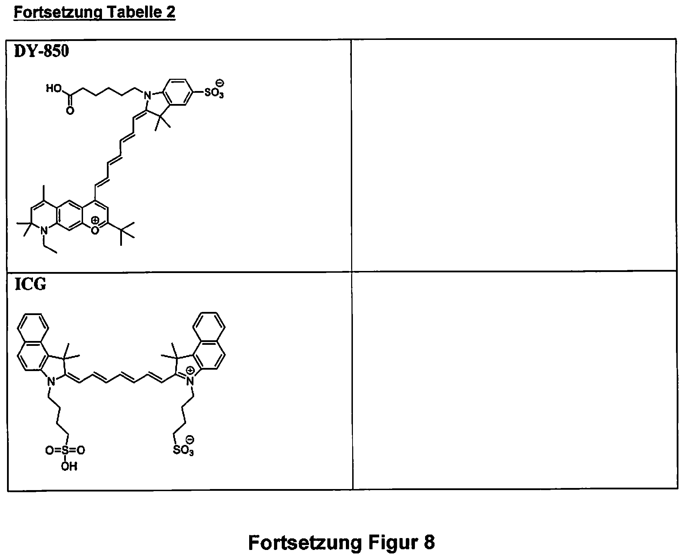

In einer bevorzugten Ausführungsform der vorliegenden Erfindung ist der mindestens eine Polymethinfarbstoff des nanostrukturierten Trägersystems ausgewähtl aus der Gruppe bestehend aus DY635, DY-680, DY-780, DY-880, DY-735, DY-835, DY-830, DY-730, DY-750, DY-850, DY-778, DY-878, DY-704, DY-804, DY-754, DY-854, DY-700, DY-800, ICG und DY-IRDYE 800CW. Weiterhin bevorzugt sind die Polymethinfarbstoffe DY-630, DY-631, DY-632, DY-633, DY-634, DY-636, DY-647, DY-648, DY-649, DY-650, DY-651, DY-652, DY-590, DY-548, DY-495 und DY-405. Hierbei handelt es sich um Polymethinfarbstoffe als Targetingeinheiten, die einen selektiven Transport in Hepatozyten- oder renale Parenchymzellen bewirken. Die allgemeinen Strukturen einer erfindungsgemäßen Hepatozyten-Targetingeinheit sowie einer erfindungsgemäßen Parenchymzell-Targetingeinheit sowie entsprechende Beispiele sind in **Tabelle 2** in **Figur 8** dargestellt. Diese Targetingeinheiten besitzen eine Selektivität für einen Zelltyp (Hepatozyten- oder renale Parenchymzellen) und können diese Zellselektivität an einen Nanopartikel oder Liposom übertragen, wenn sie mit diesem über eine chemische Bindung verbunden sind. Die Selektivität der Targetingeinheit entsteht dabei durch die Interaktion mit Influx-Transportern, welche von den Zielzellen exprimiert werden. Die Targetingeinheiten haben darüber hinaus Fluoreszenzeigenschaften im Rot- bis Inrarotbereich. Auch diese Fluoreszenzeigenschaften können das nanostrukturierte Trägersystem, genauer auf den Nanopartikel oder das Liposom übertragen werden, wodurch nicht nur die Akkumulation des Farbstoffs, sondern auch (wenn mit dem Nanopartikel bzw. dem Liposom verbunden), die Akkumulation des Nanopartikels bzw. des Liposoms im Blut und im Gewebe nachweisbar ist.

Die erfindungsgemäßen Polymethinfarbstoffe als Targetingeinheiten transportieren das nanostrukturierte Trägersystem selektiv in das Zielgewebe. Die Selektivität ist ausschlaggebend für einen erfolgreichen Transport in das "richtige" Gewebe und ausschließlich in dieses und stellt einen sehr großen Vorteil gegenüber dem Stand der Technik dar. Die Polymethinfarbstoffe dienen als Transporterliganden für gewebsspezifische Transporter. Folgende Eigenschaften sind wichtig, damit ein Polymethinfarbstoff sich als ein solcher Transporterligand eignet: (1) die Hydrophobizität und (2) die Kombination mit der spezifischen Struktur. Diese Eigenschaften sind ausschlaggebend dafür, von einem gewebsspezifischen Transporter als Ligand erkannt zu werden (Selektivität des Farbstoffs). Wird der Polymethinfarbstoff an ein Polymer oder Lipid gebunden, sodass er nach der Herstellung des Nanopartikels oder Liposoms nach außen exponiert es, überträgt er seine Selektivität auf den Nanopartikel bzw. das Liposom. Dabei kommt es nach systemischer Applikation oder lokaler Applikation zu folgenden Prozessen, die für die Selektivität des Nanopartikels bzw. Liposoms entscheidend sind:
- Vorbeiströmen des Nanopartikels oder Liposoms mit dem (mindestens einen) exponierten Polymethinfarbstoff an verschiedenen Geweben.
- Der Polymethinfarbstoff wird durch seine Hydophobizität und Struktur von gewebespezifischen basolateralen oder apikalen Influx-Transportern, erkannt und interagiert mit diesem an der Zelloberfläche.
- Die Interaktion des Polymethinfarbstoff mit dem Influx-Transpoter führt nicht zu einem direkten Transport des gesamten nanostrukturierten Trägersystems oder des Nanopartikels oder des Liposoms durch diesen Transporter, da dieser mit dem kovalent und stabil-gebundenen Nanopartikel oder Liposom ein zu hohes Molekulargewicht und Größe besitzt. Die Interaktion des Polymethinfarbstoffs mit dem Influx-Transporter führt vielmehr zu einer Akkumulation und Immobilisierung des Nanopartikels oder Liposoms an der Zelloberfläche.
- Die Akkumulation und Immobilisierung des Nanopartikels oder Liposoms an der Zelloberfläche erhöht die Interaktion zwischen Zellmembran und Nanopartikel oder Liposom, wodurch es zu einer zellulären Aufnahme (Endozytose) des Nanopartikels oder Liposoms kommt.

Die Zellselektivität ergibt sich durch die spezifische Interaktion des Polymethinfarbstoffs, welcher an den Nanopartikel oder das Liposom gekoppelt ist, und welcher von dem entsprechenden gewebsspezifischen Influx-Transporter erkannt wird. Influx-Transporter für die erfindungsgemäßen Polymethinfarbstoffe wurden für Hepatozyten und renale Parenchymzellen definiert.

Erfindungsgemäße Polymethinfarbstoffe, die spezifisch durch Influx-Transporter der basolateralen Membran von Hepatzoyten aufgenommen werden, machen den Nanopartikel für Hepatozyten spezifisch. Unter die Influx -Transporter der Hepatozyten fallen nach aktuellem Stand der Wissenschaft und FDA:

| | |
|---|---|
| | |

| Name | Gen |
|---|---|
| **OATP1 B1, OATP-C, OATP2, LST-1** | SLCO1B1 |
| **OATP1B3, OATP8** | SLCO1B3 |
| **OATP2B1** | SLCO2B1 |
| **OATP1A2** | SLCO1A2 |
| **NaDC3, SDCT2** | SLC13A3 |
| **NTCP** | SLC10A1 |
| **OCT1** | SLC22A1 |
| **OCT3** | SLC22A3 |
| **OAT2** | SLC22A7 |
| **OAT1** | SLC22A6 |
| **OAT3** | SLC22A8 |
| **PGT** | SLCO2A1 |

Liganden dieser Transporter sind insbesondere alle Polymethinfarbstoffe, die eine Struktur aufweisen, wie sie in **Tabelle 2** (Fig. 8a-e), linke Spalte, gezeigt ist.

Erfindungsgemäße Polymethinfarbstoffe, die spezifisch durch Influx-Transporter der basolateralen Membran von renalen Parenchymzellen (v.a. proximalen Tubuluszellen) aufgenommen werden, machen den Nanopartikel für diese Zelltypen spezifisch. Unter die Influx -Transporter der renalen Parenchymzellen (v.a. porimalen Tubuluszellen) fallen nach aktuellem Stand der Wissenschaft und FDA:

| Name | Gen |
|---|---|
| OCT2 | SLCO1B1 |
| OAT1 | SLCO1 B3 |
| OAT3 | SLC22A8 |
| OATP4A1 | SLCO4A1 |
| OATP4C1 | SLCO4C1 |
| OCT1 | SLC22A1 |
| OCT3 | SLC22A3 |
| PGT | SLCO2A1 |

Liganden dieser Transporter sind insbesondere alle Polymethinfarbstoffe, die eine Struktur aufweisen, wie sie in **Tabelle 2** (Fig. 8a-e), rechte Spalte, gezeigt ist.

Einer weiterhin bevorzugte Ausführungsform der Erfindung betrifft demnach ein nanostrukturiertes Trägersystem, wobei der mindestens eine Polymethinfarbstoff über mindestens einen gewebsspezifischen Transporter die Aufnahme des nanostrukturieren Trägersystems in die Zellen des Zielgewebes bewirkt. Besonders bevorzugt ist der gewebsspezifische Transporter ausgewählt aus der Gruppe bestehend aus OATP1B1, OATP-C, OATP2, LST-1, OATP1B3, OATP8, OATP2B1, OATP1A2, NaDC3, SDCT2, NTCP, OCT1, OCT3, OAT2, OAT1, OAT3, PGT, OCT2, OAT1, OATP4A1, OATP4C1

Die Begriffe "gewebsspezifischer Transporter", "Transporter" und "Influx-Transporter" werden erfindungsgemäß synonym verwendet.

Die Begriffe "nanostrukturiertes Trägersystem", "Nanopartikel" und "Liposom" werden erfindungsgemäß im Zusammenhang mit dem Transport zum und der Aufnahme in das Zielgewebe über einen gewebsspezifischen Transporter synonym verwendet.

Nach Aufnahme des nanostrukturierten Trägersystems bzw. des Nanopartikels oder des Liposoms in das Zielgewebe, kommt es zu der Freisetzung des Polymethinfarbstoffe und eines erfindungsgemäß umfassten pharmazeutischen Wirkstoffs.

Freisetzung eines Nanopartikels als Bestandteil des nanostrukturierten Trägersystems:
1. Ansäuerung des Endosoms → Destabiliserung des Nanopartikels, Abbau des Polymers durch spontane oder enzymatische Spaltung;
2. Freisetzung aktiver Substanzen (die das Endosom penetrieren können);
3. Freisetzung des Wirkstoffs, Desorption des Farbstoffs aus dem Polymer;
4. Polymerbestandteile werden verschiedenen Stoffwechselwegen zugeführt, Farbstoff wird sezemiert.

Freisetzung eines Liposoms als Bestandteil des nanostrukturierten Trägersystems:
1. Aufnahme durch Endosomen → Ansäuerung → Verschmelzen des Liposoms mit der Endosomenmembran nach Endozytose oder direkte Verschmelzung des Liposoms mit der Zellmembran;
2. Durch beide Wege kommt es zu einer direkten Freisetzung des Wirkstoffs in das Zytoplama;
3. Ist die Targetingeinheit (Polymethinfarbstoff) über eine biolabile Bindung mit dem Lipid verbunden, kann diese Bindung gespalten werden und der wird ausgeschieden. Bei Verwendung biostabiler Bindung verbleibt der Polymethinfarbstoff am Lipid. Kommt es konsekutiv zum Abbau des Lipids kann der Polymethinfarbstoff mit einem kleinen Lipidrest sezerniert werden. Es ist wahrscheinlich, dass ein Teil des Lipids mit dem Polymethinfarbstoff in die Zellmembranen eingebaut werden kann.

Das nanostrukturierte Trägersystem im Sinne der Erfindung umfasst weiterhin mindestens einen pharmazeutischen Wirkstoff. Vorzugsweise ist der mindestens ein pharmazeutischer Wirkstoff ausgewählt aus der Gruppe, bestehend aus niedermolekularen Substanzen, insbesondere Inhibitoren, Induktoren oder Kontrastmittel, sowie auch höhermolekularen Substanzen, insbesondere potentiell therapeutisch nutzbare Nukleinsäuren (z.B. short interferin RNA, short hairpin RNA, micro RNA, plasmid DNA) und Proteine (z.B. Antikörper, Interferone, Zytokine). Nachfolgende Tabelle beschreibt beispielhaft Wirkstoffe, deren spezifische Darreichung durch das nanostrukturierte Trägersystem der vorliegenden Erfindung neue therapeutische Optionen ermöglicht:

| **Wirkstoff** | **Beispiele f. Wirkstoff** | **Behandlung / Erkrankung** | **Organ / Gewebe** |
|---|---|---|---|
| Glucokortikoide | Decortin | Organtransplantation | Leber, Niere |
| Zytostatika z.B. Alkylantien | Cyclophosphoamid | Organ transplantation , Tumore | Leber, Niere |
| Antimetabolite | Methotrexat | Organtransplantation, Tumore | Leber, Niere |
| Interkalantien | Mitoxantron | Organtransplantation, Tumore | Leber, Niere |
| Antikörper | Rituximab (Anti-CD20), Daclizumab (Anti-CD25) | Organtransplantation, Tumore | Leber, Niere |
| Interferone | IFN-β, IFN-γ | Orqantransplantation | Leber, Niere |
| Phospho-Inositol-3 Kinase Inhibitoren | D-116883, AS605240, IPI-145 | Tumore, Sepsis | Leber, Niere |
| Coxibe | Celecoxib, Etoricoxib | Akutes Nierenversagen | Niere |
| JNK-Inhibitoren | CC-401, Celgene | Malaria | Leber |
| Röntgenkontrastmittel | Peritrast | Diagnose z.B. von Tumore | Leber, Niere |
| Paramagnetische Röntgenkontrastmittel | Gadopentetat-Dimeglumin (Magnevist) | Diagnose z.B. von Tumore | Leber, Niere |

Besonders bevorzugt ist der pharmazeutische Wirkstoff ein lipophiler, hydrophober, hydrophiler, amphiphiler, anionischer und/oder kationischer pharmazeutischer Wirkstoff.

Unter dem Begriff "pharmazeutischer Wirkstoff' wird erfindungsgemäß jede(s) beliebige anorganische oder organische Molekül, Substanz oder Verbindung verstanden, das (die) eine pharmakologische Wirkung aufweist. Der Begriff "pharmazeutischer Wirkstoff" wird hierin mit dem Begriff "Arzneimittel" und "Medikament" synonym verwendet.

Das nanostrukturierte Trägersystem gemäß der vorliegenden Erfindung stellt ein bisher einzigartiges, vielfach kombinierbares theragnostisches System dar, um verschiedenste Substanzen, insbesondere pharmazeutische Wirkstoffe, (z.B. hydrophile oder lipophile kleine Moleküle aber auch Nukleinsäuren) aktiv und selektiv in ein spezifisches Zielgewebe zu transportieren. Der Transport des pharmazeutischen Wirkstoffs wird durch Targetingeinheiten, Polymethinfarbstoffe, als Bestandteil des nanostrukturierten Trägersystems, die mit gewebsspezifischen Transportern auf der Zielzelle interagieren, bewirkt. Durch die Wahl des/der Polymethinfarbstoffe (DY), des/der pharmazeutischen Wirkstoffe und des/der Polymere bzw. Lipide sowie der Variation derer Parameter ist es möglich, den Nanopartikel bzw. Liposomen herzustellen, die individuell auf die jeweilige Verwendung, insbesondere den zu transportierenden pharmazeutischen Wirkstoff und/oder das Zielgewebe abgestimmt sind. Auf diese Weise ist es möglich, einen oder mehrere pharmazeutische Wirkstoffe, als Bestandteil des nanostrukturierten Trägersystems effizient in ein spezifisches Gewebe oder Zelltyp (Zielgewebe) zu transportieren und dort freizusetzen. Die pharmazeutischen Wirkstoffe können dabei solche sein, die ohne Einschluss in einen Nanopartikel oder ein Liposom lediglich eine geringe oder keine Bioverfügbarkeit haben oder eine geringe oder keine Stabilität *in vivo* aufweisen oder nur in spezifischen Organen bzw. Zellen (Zielgewebe) wirken sollen. Die Spezifität und Akkumulation des nanostrukturierten Trägersystems (Nanopartikel oder Liposom) und/oder Bestandteilen hiervon, wie Polymere, Lipide oder pharmazeutische(n) Wirkstoff(e) in dem Zielgewebe, kann über die Fluoreszenzeigenschaften im Rot- bis Infrarotbereich des/der nicht toxischen Polymethinfarbstoffs/e überprüft und verfolgt, also detektiert, werden.

"Zielgewebe" sind alle Gewebe, Organe oder Zellen, in die der Transport eines nanostrukturierten Trägersystems und/oder Bestandteilen hiervon, vor allem eines pharmazeutischen Wirkstoffs, möglich und sinnvoll ist. Zielgewebe sind insbesondere alle Gewebe, Organe oder Zellen, in die der Transport eines oder mehrerer pharmazeutischer Wirkstoffe möglich und sinnvoll ist, beispielsweise zur Behandlung oder Diagnose einer Erkrankung. Zielgewebe im Sinne der Erfindung sind Leber, Niere und Tumore mit Ursprung in diesen Geweben, beispielsweise hepatozelluläre Karzinome oder Hypernephrome. Die Begriffe "Zielgewebe", "Zielzelle", "Zellen eines Zielgewebes" und "Organ" werden hierin synonym verwendet.

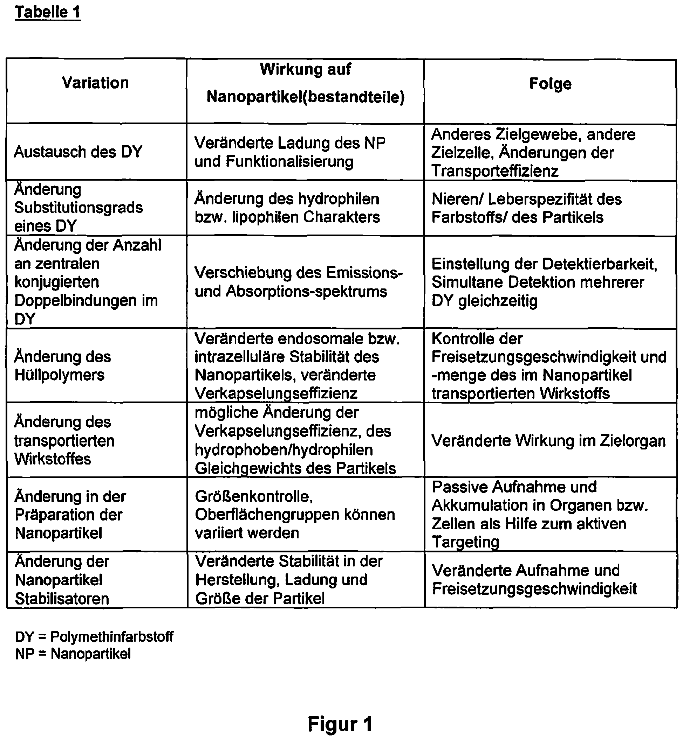

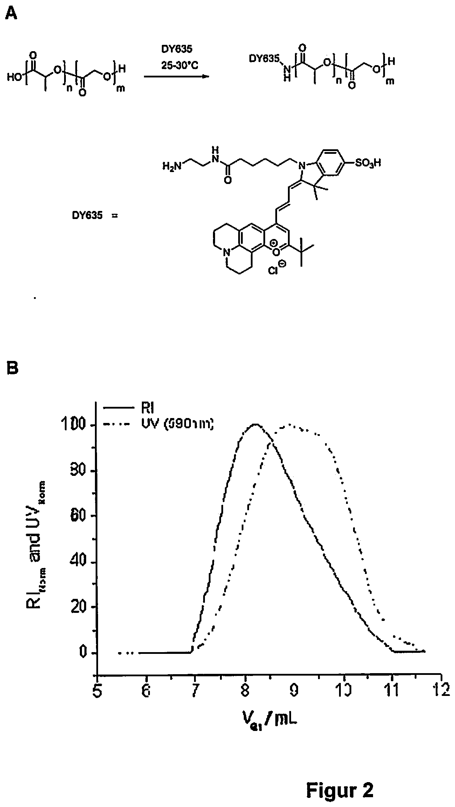

Durch die Konjugation der erfindungsgemäßen Polymethinfarbstoffe (im folgenden DY) an Polymere oder Lipide, werden funktionalisierte Polymere (z.B. DY-PLGA, DY-PLA, DY-PCL) bzw. funktionalisierte Lipide hergestellt. Anschließend werden diese zur Nanopartikel- bzw. Liposomherstellung, vorzugsweise mittels einfacher oder doppelter Emulsionstechnik oder Präzipitationstechnik, verwendet. Dabei ist es möglich, die Nanopartikel bzw. Liposomen individuell auf eine jeweilige Fragestellung abzustimmen. Die vielfältigen Möglichkeiten sind beispielhaft **Tabelle 1** (Figur 1) aufgeführt. Die Polymethinfarbstoffe sind mit einer Vielzahl verschiedener Polymere bzw. Lipide konjugierbar, sodass durch die spezielle Kombination von Polymethinfarbstoff mit einem Lipid oder Polymer hochselektive nanostrukturierte Trägersysteme bereitgestellt werden können. Die Synthese funktionalisierter Polymere wird in **Figur 2** schematisch dargestellt und in **Beispiel 1** detailliert erläutert. Die Herstellung erfindungsgemäßer funktionalisierter Polymere oder Lipiden, Nanopartikel und Liposome sowie der Einschluss pharmazeutischer Wirkstoffe kann nach herkömmlichen Verfahren aus dem Stand der Technik erfolgen. In den Beispielen und Figuren der vorliegenden Erfindung werden bevorzugte Herstellungsverfahren offenbart.

Eine weitere Offenbarung betrifft eine pharmazeutische Zusammensetzung, die ein nanostrukturiertes Trägersystem gemäß der Erfindung sowie geeignete Hilfs- und Zusatzsstoffe enthält.

Unter "Hilfs-und Zusatzstoffen" ist jede pharmakologisch verträgliche und therapeutisch sinnvolle Substanz zu verstehen, die kein pharmazeutischer Wirkstoff ist, jedoch zusammen mit dem pharmazeutischen Wirkstoff in der pharmazeutischen Zusammensetzung formuliert werden kann, um qualitative Eigenschaften der pharmazeutischen Zusammensetzung zu beeinflussen, insbesondere zu verbessern. Bevorzugt entfalten die Hilfs- und/oder Zusatzstoffe keine oder im Hinblick auf die beabsichtigte Behandlung keine nennenswerte oder zumindest keine unerwünschte pharmakologische Wirkung. Geeignete Hilfs- und Zusatzstoffe sind beispielsweise pharmazeutisch verträglichen anorganischen oder organischen Säuren, Basen, Salze und/oder Puffersubstanzen. Beispiele für anorganische Säuren sind Salzsäure, Bromwasserstoffsäure, Salpetersäure, Schwefelsäure und Phosphorsäure, wobei insbesondere Salzsäure und Schwefelsäure bevorzugt sind. Beispiele für geeignete organische Säuren sind Äpfelsäure, Weinsäure, Maleinsäure, Bernsteinsäure, Essigsäure, Ameisensäure und Propionsäure und insbesondere bevorzugt Ascorbinsäure, Fumarsäure und Zitronensäure. Beispiele für pharmazeutisch verträgliche Basen sind Alkalihydroxide, Alkalicarbonate und Alkali-Ionen, bevorzugt Natrium. Mischungen dieser Substanzen können insbesondere zur Einstellung und Pufferung des pH Wertes benutzt werden. Bevorzugte Puffersubstanzen im Sinne der Erfindung sind weiterhin PBS, HEPES, TRIS, MOPS sowie weitere physiologisch verträgliche Puffersubstanzen. Weitere geeignete Hilfs- und Zusatzstoffe sind Lösungs- oder Verdünnungsmittel, Stabilisatoren, Suspensionsvermittler, Konservierungsmittel, Füllstoffe, und/oder Bindemittel sowie weitere im Stand der Technik bekannte übliche Hilfs- und Zusatzstoffe. Die Auswahl der Hilfsstoffe sowie die einzusetzenden Mengen derselben hängt von dem pharmazeutischen Wirkstoff und der Art der Verabreichung ab. Pharmazeutische Zusammensetzungen der vorliegenden Erfindung werden bevorzugt parenteral, insbesondere intravenös verabreicht. Für alle parenteralen Applikationen eignen sich Zubereitungen in Form von Suspensionen und Lösungen sowie leicht rekonstituierbare Trockenzubereitungen.

Die Herstellung einer pharmazeutischen Zusammensetzung kann durch jedes beliebige im Stand der Technik bekannte Verfahren erfolgen.

Die Dosierung der Komponenten einer erfindungsgemäßen pharmazeutischen Zusammensetzung unterliegt verschiedenen Faktoren, beispielsweise der Art des pharmazeutischen Wirkstoffs, der Erkrankung, des Zustands des Patienten (Säugetier, vorzugsweise Mensch), dem die erfindungsgemäße pharmazeutische Zusammensetzung verabreicht wird und der Art der Verabreichung, z.B. parenteral, intravenös oder auf andere Weise appliziert werden soll. Dem Fachmann sind solche Parameter bekannt, die Bestimmung der Dosierungen unterliegt somit seinem allgemeinen Fachwissen.

Eine weitere Offenbarung betrifft die Verwendung eines nanostrukturieren Trägersystems oder einer pharmazeutischen Zusammensetzung gemäß der Offenbarung zum aktiven und selektiven Transport des nanostrukturieren Trägersystems oder der pharmazeutischen Zusammensetzung in ein Zielgewebes, wobei der Transport durch den mindestens einen Polymethinfarbstoff als Targetingeinheit bewirkt wird. Besonders bevorzugt bewirkt der mindestens eine Polymethinfarbstoff über mindestens einen gewebsspezifischen Transporter die Aufnahme des nanostrukturierten Trägersystems oder der pharmazeutischen Zusammensetzung in die Zellen des Zielgewebes. Insbesondere bevorzugt ist die Akkumulation des nanostrukturieren Trägersystems und/oder seiner Bestandteile in einem Zielgewebe mittels Floureszenzeigenschaften des mindestens einen Polymethinfarbstoffs detektierbar ist. Als Bestandteile des nanostrukturierten Trägersystems (Nanopartikel oder Liposom) sind -neben dem mindestens einen Polymethinfarbstoffmindestens ein Polymer, mindestens ein Lipid und/oder mindestens ein pharmazeutischer Wirkstoff zu verstehen.

Eine weitere Offenbarung betrifft ein nanostrukturieres Trägersystem oder eine pharmazeutische Zusammensetzung gemäß der Offenbarung zur Verwendung als Arzneimittel.

Das nanostrukturieres Trägersystem gemäß der Erfindung oder die pharmazeutische Zusammensetzung gemäß der Offenbarung ist zur Verwendung für die Behandlung von Erkrankungen der Leber und/oder Niere, vorzugsweise Infektionserkrankungen mit Schädigung der Leber und/oder Niere, beispielsweise Malaria und Hepatitis C, Leberversagen beispielsweise Medikamenten-induziertes Leberversagen und fulminantes Leberversagen, Leberzirrhose, beispielsweise Alkohol-induzierte Leberzirrhose, Stoffwechselerkrankungen der Leber, beispielsweise Morbus Wilson und Morbus Meulengracht, exkretorische Dysfunktionen der Leber, Lebertumore, primäre Lebertumore, beispielsweise hepatozelluläre Karzinome, Angiosarkom und Hepatoblastom, Nierentumore, primäre Nierentumore, beispielsweise Klarzellkarzinom, papilläres Karzinom und chromophobes Karzinom, Nephritiden, chronisches und akutes Nierenversagen sowie Erkrankungen die eine konsekutive Schädigung der Leber und/oder der Niere verursachen, beispielsweise Sepsis.

Die erfindungsgemäßen nanostruktuierten Trägersysteme und Targetingeinheiten, insbesondere Polymethinfarbstoffe, eröffnen eine einzigartige Möglichkeit die Diagnose in einem Molekül mit der Therapie zu verbinden. So lassen sich Voraussagen über die Effektivität der Therapie durch die Aufnahme der freien Targetingstruktur vorhersagen, aber auch die Therapie mit derselben Targetingstruktur am Nanopartikel oder Liposom gebunden überwachen und kontrollieren. Durch die hohe Flexibilität der Targetingstruktur in der Linker-Region kann die Targetingeinheiten an verschiedenste Lipide und Polymere chemisch gebunden werden. Durch die chemische Struktur der Targetingeinheit ist diese weiterhin im Gegensatz zu biologischen Targetingeinheiten (z.B. Antikörper oder Peptide) sehr stabil und für chemische Reinigung und Analyse zugänglich. Dadurch ist eine hohe Reproduzierbarkeit und Kontrollierbarkeit in der Synthese möglich. Durch die Eigenschaft der Targetingeinheit als Ligand gewebsspezifischer Transporter kann diese nach Desorption aus dem Polymer *in vivo* ausgeschieden werden, wodurch die intrazelluläre Akkumulation und Toxizität vermieden wird. Durch aktuelle bildgebende Entwicklungen im Bereich der multispektralen optoakustischen Tomographie, lässt sich die Targetingeinheit direkt nachweisen. Weiterhin können aber auch erfindungsgemäß Kontrastmittel für die computergestützte Röntgentomographie oder Magnetresonanztomographie in die Nanopartikel oder Liposomen eingeschlossen werden, wodurch sie ebenfalls lokalisiert werden können.
Ein derartiges vielfältiges und zell-spezifisches System, welches die Diagnose und Therapie über einen im rot- bis Infrarot-bereich fluoreszierenden Farbstoff als Targetingeinheit verbindet, welche wiederum durch ihre Selektivität zu Biotrasportern auch sehr Effektivität von Leber und Niere ausgeschieden wird ist bisher einzigartig.

Die Erfindung wird ferner anhand von Figuren beispielhaft illustriert:
**Figur 1** zeigt eine Übersicht der möglichen Variationen eines erfindungsgemäßen Nanopartikels und deren Einfluss auf die physikochemischen Eigenschaften der Nanopartikel selbst und auf die biologischen Folgen **(Tabelle 1).**
**Figur 2** stellt schematisch die Funktionalisierung der erfindungsgemäßen Polymere dar. **A:** Synthese des funktionalisierten PLGA Polymers durch EDC Kopplung des Polymethinfarbstoffs DY635 an die Carbonsäure-Endgruppe des PLGA zu DY635-PLGA-NP (oder auch als DY635-PLGA bezeichnet; beide Begriffe werden in der vorliegenden Erfindung synonym verwendet). B: SEC Elugramm der funktionalisierten PLGA Polymere mit UV und IR Detektor. Synthese und Funktionalisierung werden ferner in Beispiel 1 detailliert beschrieben.

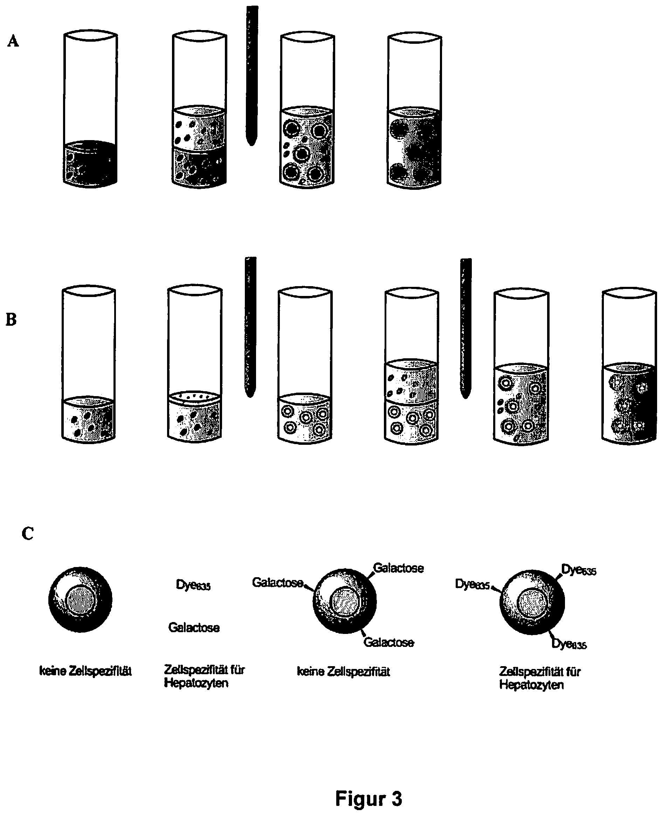

**Figur 3** zeigt den Aufbau und die Herstellung erfindungsgemäßer Nanopartikel. Die einzelnen Ultraschallschritte sind durch graue Nadeln (Pfeile) gekennzeichnet. Eine detaillierte Beschreibung findet sich ferner in Beispiel 2. **A:** Aufbau der Nanopartikel und deren Herstellung mittels einfacher Emulsionstechnik. In dunkelgrau dargestellt ist das hydrophobe Polymer, mit in mittelgrau dargestelltem hydrophobem Wirkstoff, in hellgrau dargestellt das Surfaktant (Tensid) in Wasser. **B:** Aufbau der Nanopartikel und deren Herstellung mittels doppelter Emulsionstechnik. In dunkelgrau dargestellt ist das hydrophobe Polymer, mit in weiß dargestelltem hydrophilem Wirkstoff. Die obere hellgraue Schicht ist dann wiederum Wasser mit Tensid. **C:** Übersicht (Querschnitte) der möglichen Variationen eines erfindungsgemäßen Nanopartikels und deren Einfluss auf die physikochemischen Eigenschaften der Nanopartikel selbst und auf die biologischen Folgen **(Tabelle 1).** In schwarz dargestellt ist das hydrophobe Polymer oder Lipid, in grau dargestellt ein möglicher pharmazeutischer Wirkstoff, Galactose und DY635 als Transporter für die Zellspezifische Aufnahme in Hepatozyten, wobei nur der erfindungsgemäßen Nanopartikel die Zellspezifität beibehält.

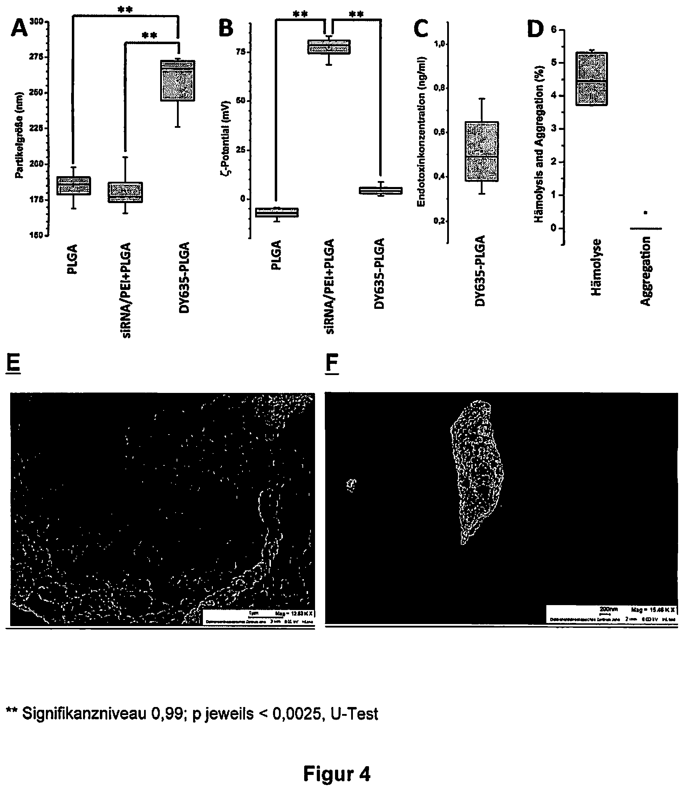

**Figur 4** zeigt die Ergebnisse der Charakterisierung einer Auswahl erfindungsgemäßer Nanopartikel. Die Boxplots umfassen das 0,25 bis zum 0,75 Quantil. Der Median ist als horizontaler Balken eingetragen, der Mittelwert als Quader. Die Whiskers zeigen jeweils den größten bzw. kleinsten Wert an. **A:** Die Größe der PLGA-Nanopartikel unterscheidet sich nicht von den siRNA/PEI beladenen PLGA-Nanopartikeln (etwa 180 nm). Die DY635-PLGA-Nanopartikel sind dahingegen signifikant größer (etwa 260 nm). **B:** Das z-Potential von PLGA-Nanopartikel ist leicht negativ. Durch die Verwendung von DY635-PLGA-Nanopartikel schwenkt das Potential in das schwach positive z-Potential (keine Signifikanz). Durch die Beladung mit den siRNA/PEI Polyplexen (siRNA/PEI+PLGA-Nanopartikel) ändern sich das z-Potential signifikant und wird stark positiv (+ 76 mV). **C:** Zur Bestimmung der Endotoxinmenge wurden Nanopartikel(NP)-Lösungen der Konzentration 25 mg NP/ml, wie sie auch *in vivo* verwendet werden, untersucht. Die Endotoxinbelastung schwankte zwischen den Proben zwischen 0,4 und 0,6 ng/ml. Der Wert lag jedoch immer unterhalb des Grenzwerts der FDA (2,5 ng/ml). **D:** NP-Lösungen von 25 mg/ml werden auch für den Hämolyse- und Aggregationsassay verwendet. Für den Assay wurden DY635-PLGA-Nanopartikel verwendet, wie sie auch in den *in vivo* Versuchen verwendet werden. Eine weitere Beschreibung hierzu findet sich in Beispiel 3.

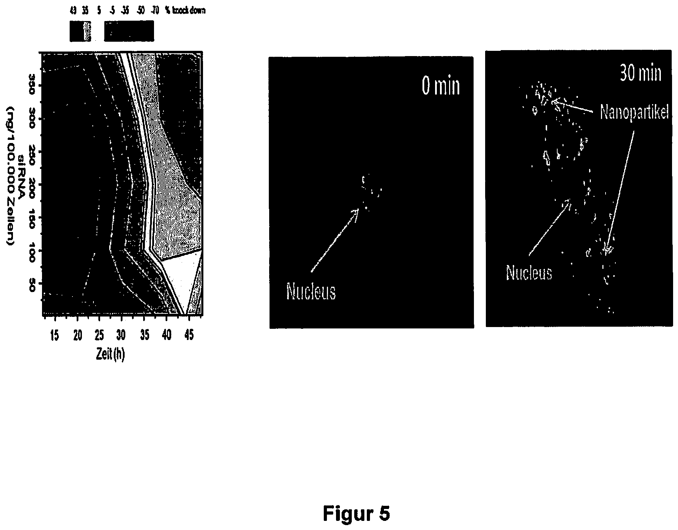

**Figur 5** zeigt die Aufnahmekinetik und Charakterisierung des RNAi in Hepa1-6 Zellen in *vitro.* **A:** Diagramm (Heatmap), welches das zeit- und konzentrationsabhängige RNAi beschreibt. Auf den Achsen ist die Zeit in Stunden (h) gegen die siRNA-Konzentration (ng/100.000 Zellen) abgebildet. Die HMGCR-Expressionsänderung gegenüber unbehandelten Kontrollen ist in Graustufen skaliert in Prozent abgebildet (Skala über dem Bild). Für die Punkte in der Heatmap werden siRNA-Konzentrationen von 1, 5, 10, 25, 50, 100, 200, 400 ng / 100.000 Zellen verwendet und nach 12, 16, 24, 32, 40 oder 48 h untersucht. Für jeden Zeitpunkt wurden drei unabhängige Replikate generiert. Das Ergebnis wurde anschließend gegen das HMGCR-Genexpressionsniveau unbehandelter Hepa1-6 Zellen relativiert und mit Hilfe der HPRT-Genexpression normalisiert. **B:** Aufnahme der Nanopartikel in Hepa1-6 Zellen nach 0 und 30 Minuten (min). DY635 wird im Cy5-Channel am LSM visualisiert. Die Zellkerne werden nach Waschen und Fixieren der Zellen DAPI gefärbt. Eine weitere Beschreibung hierzu findet sich in Beispiel 4.

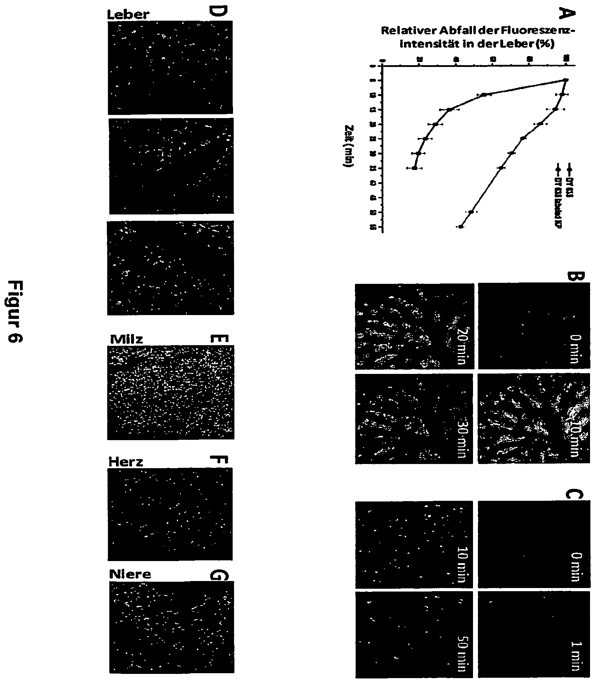

**Figur 6** stellt die Organspezifität und Kinetik eines erfindungsgemäßen Nanopartikels in Leber, Niere, Milz und Herz dar. **A:** Vergleich der Abklingkinetik DY635 vs. DY635-PLGA-NP. Mittelwerte aus 3 ROIs in der Leber. Fehlerbalken stellen die SEM dar. **B+C:** Überlagerung der Bilder von Cy5 (DY635)- (**B** hellgrau nach weiß, **C** hellgrau) und DAPI (Hintergrund)-Channel am IVM zu verschiedenen Zeitpunkten. **D-G:** 5 µm Organschnitte 10 Minuten nach DY635-PLGA-NP-Injektion. Auf den Bildern wird DY635-PLGA-NP bzw. DY635 (Cy5-Channel, grün im Bild) und die Zellkerne (DAPI gefärbt, rot im Bild) überlagert. **F,G:** Hier sind die Färbungen zusätzlich mit der, im Phasenkontrast visualisierten, Leberstruktur (blau im Bild) überlagert. Eine weitere Beschreibung findet sich in Beispiel 5.

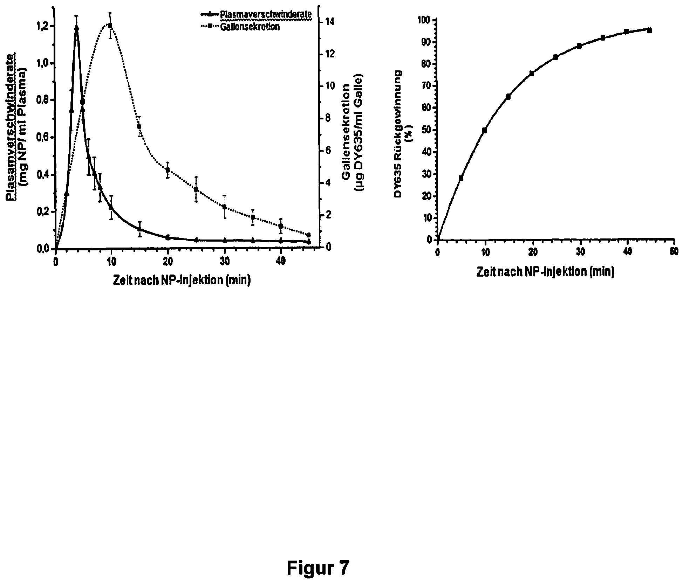

**Figur 7** stellt die Sekretionsroute eines erfindungsgemäßen Nanopartikels dar. Sekretionsroute von DY635-PLGA-NP. **A:** Für die Berechnung der Plasmaverschwinderate wird eine Standardkurve in unbehandeltem Plasma mit DY635-PLGA-Nanopartikel erstellt. Für die Gallensekretion wurde eine Standardreihe mit DY635 in Galle verwendet. **B:** zeigt das prozentualen DY635 "recovery" (Wiedergewinnung) in der Galle. Die Messpunkte entstanden rechnerisch, basierend auf den Daten aus A. Die Kurve wurde mittels OriginPro 8.5, QucikFit: Exponential Decay mit Offset approximiert.
**Figur 8** zeigt erfindungsgemäße Targetingeinheiten, und zwar Polymethinfarbstoffe. Figur 8 zeigt die allgemeine Struktur für eine Hepatozyten-Targetingeinheit und die allgemeine Struktur für eine renale Parenchymzell-Targetingeinheit mit Angabe des Linkers zum Polymer bzw. Lipid sowie Beispiele für solche Hepatozyten-Targetingeinheiten und Parenchymzellen-Targetingeinheiten **(Tabelle 2).** Die Targetingeinheiten besitzen eine Selektivität für einen Zelltyp (Hepatozyten- oder renale Parenchymzellen) und können diese Zellselektivität an einen Nanopartikel oder Liposom übertragen, wenn sie mit diesem über eine chemische Bindung verbunden sind. Die Selektivität der Targetingeinheit entsteht dabei durch die Interaktion mit Influx-Transportern, welche von den Zielzellen exprimiert werden. Die Targetingeinheiten haben darüber hinaus Fluoreszenzeigenschaften im Rot- bis Inrarotbereich. Auch diese Fluoreszenzeigenschaften können das nanostrukturierte Trägersystem, genauer auf den Nanopartikel oder das Liposom übertragen werden, wodurch nicht nur die Akkumulation des Farbstoffs, sondern auch (wenn mit dem Nanopartikel bzw. dem Liposom verbunden), die Akkumulation des Nanopartikels bzw. des Liposoms im Blut und im Gewebe nachweisbar ist.

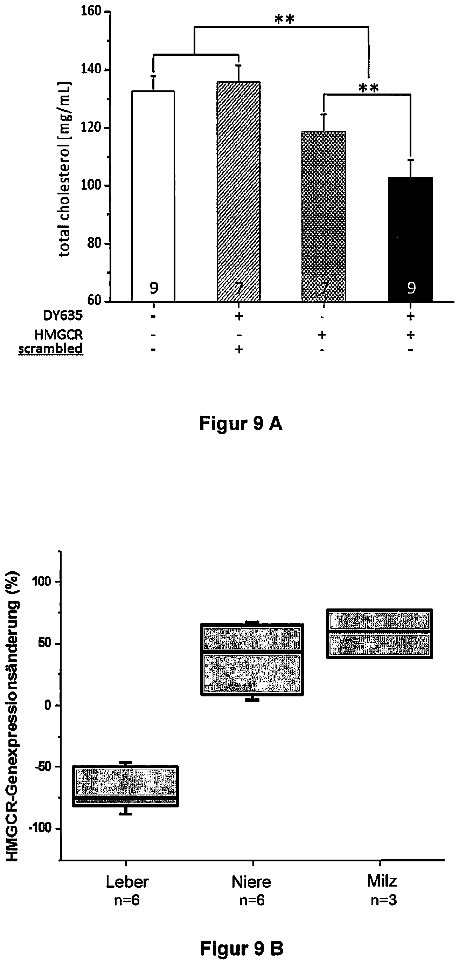

**Figur 9** zeigt die Wirksamkeit der erfindungsgemäßen nanostrukturierten Trägersysteme in dem Transport eines pharmazeutischen Wirkstoffs. **A** Plasmacholesterolspiegel nach zwei Injektionen der nanostrukturierten Trägersysteme, welche eine siRNA gegen die HMGCR transportieren bzw. nach zwei Injektionen einer Kontrollsubstanz. Die Abbildung zeigt einen Median Bar-Plot, Fehlerbalken beschreiben den mittleren Fehler der Standardabweichung, die Nummern in den Balken beschreiben die Tierzahl in jeder Gruppe, die Signifikanzen wurden durch einen zweiseitigen U-Test ermittelt, ** Signifikanzniveau 0,01.
**Figur 9** **A:** Die Ergebnisse zeigen, dass es durch den beschriebenen Ansatz möglich ist, die plasma-cholesterin-konzentration signifikant zu senken. Dabei zeigte das organspezifische nanostrukturierte Trägersystem die stärkste Wirkung. Aus **Figur 9** **B** wird deutlich, dass durch das organspezifische nanostrukturierte Trägersystem ein organspezifischer und starker Effekt in Hepatozyten erzielt wurde. Das unspezifische nanostrukturierte Trägersystem hingegen zeigt keine spezifische und schwächere Herabregulation der HMGCR.

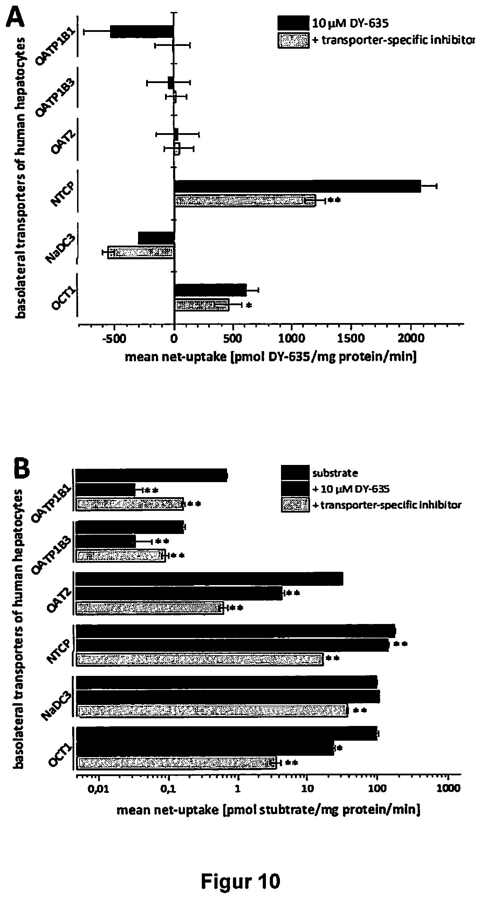

**Figur 10** zeigt eine erfindungsgemäße Interaktion eines erfindungsgemäßen Polymethinfarbstoffs als Targetingeinheit, DY-635, mit einem humanen basolateralen Transporter der Hepatozyten. **A, B** zeigen Bar-Plot des Mittelwerts, Fehlerbalken beschreiben den mittleren Fehler der Standardabweichung, alle Versuche wurden 6 Mal durchgeführt. Die Signifikanzen wurden durch einem zweiseitigem U-Test ermittelt, **Signifikanzniveau 0,01, * Signifikanzniveau 0,01.

Die Erfindung wird nachfolgend anhand von Beispielen demonstriert, ohne hierauf beschränkt zu sein.

### Beispiele

### Beispiel 1: Synthese funktionalisierter Polymere

Die synthetisierten Nanopartikel basieren auf dem hydrophoben Polymer *poly(lactic-co-glycolic acid)* (PLGA), welches biokompatibel und bioabbaubar ist. Dieses Polymer kann aufgrund seiner aktiven Carbonsäure-Gruppe ("acid terminated") durch Kopplungsreagenzien wie EDC (1-ethyl-3-(3-dimethylaminopropyl)carbodiimid) an einen Amin-funktionalisierten Farbstoff kovalent gebunden werden. Hier wurde der Polymethinfarbstoff DY635 verwendet (siehe **Figur 2**). Dabei wurde jede 100ste Polymerkette funktionalisiert. Die Polymere wurden anschließend mittels Dialyse vom freien Farbstoff DY635 getrennt und mittels Präzipitation aufgereinigt. Die Charakterisierung erfolgte dabei durch Größenausschlusschromatographie (SEC), bei der ein UV/Vis und ein RI (*refractive index*) Detektor miteinander kombiniert wurden. Die graphische Darstellung der Synthese sowie ein SEC Elugramm sind in den **Figur 2** dargestellt.

### Beispiel 2: Herstellung von Nanopartikeln

Nach der Funktionalisierung der Polymere (Beispiel 1) wurden mittels einfacher (A) und doppelter (B) Emulsion Nanopartikel hergestellt. Dabei wurde hochfrequenter Ultraschall verwendet, der mit Hilfe von Oberflächen-aktiven Substanzen (Tenside, Surfactant), hier Polyvenylalkohol (PVA) die Bildung von nanoskaligen Partikeln begünstigt. Die hydrophoben Polymere wurden dafür in Ethylacetat, ein mit Wasser nicht mischbares Lösungsmittel, gelöst (25 mg/ml) gelöst. Als Surfactant (Tensid) wurde 0,3% PVA (Polyvinylalkohol) in Reinstwasser verwendet, wobei die gesamte Polymerkonzentration 2,5 mg/ml betrug. Die Polymersuspension in Ethylacetat wurde zu Wasser mit Surfactant gegeben und durch Ultraschall Nanopartikel gebildet (A). Sollen hydrophile Substanzen eingeschlossen werden, wird zuerst die hydrophile Substanz in Wasser gelöst zu dem Polymer in Ethylacetat gegeben und geultraschallt. Anschließend wird dazu Wasser mit Surfactant gegeben und wieder durch Ultraschall Nanopartikel gebildet. Die Ergebnisse der Emulsionstechnik sind in **Figur 3** dargestellt.

Die so hergestellten Nanopartikel mit einem Durchmesser von etwa 200 nm wurden anschließend unter einem Luftstrom gerührt, bis das gesamte organische Lösungsmittel (Ethylacetat) verdampft war und die Partikel damit stabil in Wasser waren. Um das überschüssige Tensid zu entfernen, wurden die Nanopartikel mindestens zweimal gründlich mit Reinstwasser gewaschen. Dies kann durch Vortexen und Inkubation im Ultraschallbad unterstützt werden. Abschließend wurden die Partikel lyophilisiert und deren Masse bestimmt.

### Beispiel 3: Charakterisierung der Nanopartikel

Es wurden Nanopartikel aus DY635-konjugiertem PLGA (DY635-PLGA-NP) mit konstanten Parametern hergestellt und reproduziert. Die hierzu verwendeten Assays werden im Folgenden aufgeführt:
- Größe: Messung der Größe der verschiedenen, in entionisiertem Wasser gelösten, nanostrukturierten Trägersysteme durch dynamische Lichtstreuung (beispielsweise Zetasizer (Malvern Instruments GmbH)) oder elektronenmikroskopische Aufnahmen.
- Form: Bestimmung der Form durch elektronenmikroskopische Aufnahmen.
- Ladung: Messung der verschiedenen, in entionisiertem Wasser gelösten, nanostrukturierten Trägersysteme am Zetasizer (Malvern Instruments GmbH) durch Bestimmung der elektrophoretischen Signals (ζ-Pοtential, Oberflächenladung).
- Endotoxine: Endotoxinmessung durch LAL chromogenic Assay nach Guilfoyle, D.E. et al., Evaluation of a chromogenic procedure for use with the Limulus lysate assay of bacterial endotoxins in drug products. J Parenter Sci Technol, 1985. 39(6): S. 233-6
- Hämolyse: Messung der Hämoglobinkonzentration von Erythrozythen, die mit den Partikeln in physiologischme Puffer für 1 h inkubiert wurde. Bei Schädigung der Erythrozytenmembran steigt die messbare Hämoglobinkonzentration im Überstand.
- Aggregation: Messung der Absorption von Erythrozyten, die mit den Polymeren in physiologischem Puffer inkubiert wurden. Proben mit Zellaggregaten zeigen eine geringere Absorption, als homogen verteilte, nicht aggregierte Zellen.

Die Ergebnisse sind in Figur 4 dargestellt. **A:** Größe, **B:** Ladung, **C:** Endotoxinbelastung. Für die Größenmessung wurde die Dynamische Lichtstreuung verwendet, die Ladung wurde mittels ζ-Potential bestimmt. **D:** Weiterhin wurde gezeigt, dass DY635-PLGA-NP keine lysierenden Eigenschaften im Blut besitzen und diese nicht zur Aggregation von Erythrozyten führen. **E und F:** In elektronennmikroskopische Aufnahmen (SEM) zeigen die Partikel eine runde bzw. sphärische Form., sowohl unbeladen und ohne Targeting (E) als auch mit Targeting und beladen (F).

### Beispiel 4: Induzieren von "Drug-Associated Effects" durch RNAi und Aufnahme in vitro ("Proof of Concept")

**Durchführung zu** **Figur 6** **A:** Hepa1-6 Zellen wurden unter Standardkulturbedingungen (37 °C, 5% CO2, DMEM 4,5g/l Glukose, 10% hitzeinaktiviertes fetales Rinderserum, 1% Penicillin/Streptomycin) in 6-Well-Platten kultiviert (100.000 Zellen/9,6 cm²). Nach 24 Stunden wurden verschiedene Konzentrationen des nanostrukturierten Trägersystem, welches wie in Beispiel 7 B hergestellt wurde, zu den Wells gegeben und für verschiedene Zeiträume inkubiert (Konzentrationen und Inkubationszeiten sind in Figur 5 A ablesbar). Nach der Inkubationszeit wurden die Zellen mit *Hank's Balanced Salt Solution* (HBSS) gewaschen und mit RLT-Puffer (Qiagen GmbH), dem 1% β-Mercaptoethanol beigemengt wurde, lysiert. Aus dem Lysat wurde die mRNA isoliert und in der RT-qPCR analysiert. Die Werte wurden anschließend auf das Hypoxanthin-Guanin-Phosphoribosyltransferase Expressionsniveau normalisiert und das HMGCR-Expressionsniveau (HMGCR: 3-Hydroxy-3-Methylglutaryl-Coenzym-A-Reduktase oder HMG-CoA) zu nicht transfizierten Hepa1-6 Zellen verglichen.

**Durchführung zu** **Figur 6** **B:** Hepa1-6 Zellen wurden unter Standardkulturbedingungen auf Chamber Slides (Nunc, Thermo Scientific GmbH) kultiviert (5 000 Zellen/1,5 cm²). Nach 24 Stunden wurden die Zellen mit 100 µg/ml (Endkonzentration) DY-635 modifiziertem nanostrukturierten Trägersystem (Hergestellt wie in Beispiel 7 B beschrieben) versetzt. Nach 30 Minuten Inkubation bei Standardkulturbedingungen mit dem nanostrukturierten Trägersystem wurden die Zellen mit HBSS gewaschen und für 15 min mit 5% Formalin (pH 7) fixiert. Anschließend wurden die Objektträger mit den Zellen gewaschen und die Zellkerne mit DAPI gefärbt. Zur Auswertung durch *Laser Scanning Microscopy* wurden die Zellen mit VectaShield (Vector Labs, Inc.) befeuchtet und mit einem Deckglas versiegelt. Das nanostrukturierte Trägersystem wurde durch die Modifikation mit DY-635 bei 633 nm (Exzitation) detektiert, die Zellkerne wurden bei 460 nm (Exzitation) visualisiert.

Die Ergebnisse sind in Figur 5 dargestellt. **A:** Durch siRNA-Transfektion in Hepa1-6 Zellen konnte die HMGCR-Genexpression um bis zu 70 % herunter regulieren. HMGCR (3-Hydroxy-3-Methylglutaryl-Coenzym-A-Reduktase oder HMG-CoA) verkörpert das Schlüsselenzym eines zentralen Stoffwechsels - der Cholesterolbiosynthese. Das in diesem Experiment gezeigte Herabregulieren dieses Stoffwechselgens zeigt die Effektivität des erfindungsgemäßen Wirkstofftransports durch die nanostrukturierten Trägersysteme. Ferner hat ein erhöhter Plasma-cholesterinspiegel bei der Entstehung von Arteriosklerose eine zentrale Bedeutung. Diese hier gezeigte Behandlungsmethode unter Verwendung der nanostrukturierten Trägersysteme der Erfindung stellt eine interessante Alternative zur herkömmlichen Behandlung mit Statinen dar, zudem stellt dies den ersten Schritt in Richtung Gen-Transport für Menschen mit einem erblich bedingten erhöhten Cholesterinspiegel (familiäre Hypercholesterinämie) dar. **B:** zeigt, dass DY635-PLGA-NP innerhalb von 30 Minuten von Hepa1-6 Zellen (murine Hepatomazelllinie) aufgenommen werden. Eine derartig schnelle und intensive Aufnahme eines Nanopartikels ist im Stand der Technik bislang nicht beschrieben.

### Beispiel 5: In vivo-targeting: Organspezifität und Beschreibung der Sekretionsroute

Die Herstellung des nanostrukturierten Trägersystems für dieses Experiment erfolgte wie in Beispiel 2 (B) beschrieben. Für die Injektion wurde das gefriergetrocknete nanostrukturierte Trägersystem unter Zuhilfenahme eines Orbitalmixers und Ultraschallbads in einer sterilen, 5 %igen Glukoselösung (Glucosteril G5, Fresenius SE&Co KGaG) gelöst.

**Durchführung,** (Figur 6 **A**): Mäuse oder Ratten wurden venös katheterisiert (Vena jugularis). Anschließend wurden die Leber *ex situ* auf einem intravitatmikroskop präpariert. Anschließend wurde DY-635 (13 pmol/g Körpergewicht (KG)) oder das nanostrukturierte Trägersystem, welches eine DY-635 Modifikation trägt, (6,5 µg/ g KG) venös injiziert und die spezifische Fluoreszenz des DY-635 bei 633 nm in der Leber über die Zeit gemessen und verschiedene *Region of Interests* (ROIs) in den aufgenommenen Bildern über die Zeit quantifiziert. In Figur 6 sind repräsentative Bilder der Messung des DY-635 (Figur 6 **B**) oder des DY-635 modifizierten nanostrukturierten Trägersystems (Figur 6 **C**) dargestellt. DY-635 oder das DY-635 modifizierte nanostrukturierte Trägersystem wurde durch die Fluoreszenz des DY-635 bei 633 nm dargestellt. Die Leberstruktur wurde durch die Autofluoreszenz des NADH/NADH+ bei 450 nm dargestellt. Um die Organspezifität darzustellen, wurde männlichen Mäusen 6,5 µg des DY-635 modifizierten Trägersystem pro g KG über einen zentralvenösen Katheter injiziert. 10 Minuten nach Injektion wurde das Tier schmerzlos euthanasiert und die Organe entnommen und für die histologische Aufarbeitung Kryopräpariert. Anschließend wurden am Kryotom 5 µm dicke Schnitte der Organe angefertigt und diese mit DAPI gegengefärbt. Anschließend wurden alle Organe bei gleichen Einstellungen hinsichtlich der DAPI-gefärbten Zellkerne (bei 430 nm) und hinsichtlich des Nanopartikels (bei 633 nm) untersucht.

Die **Ergebnisse** sind in Figur 6 dargestellt. **C:** Die Nanopartikel DY635-PLGA-NP werden bereits nach 1 Minute in Hepatozyten aufgenommen (Kopfsteinpflaster-artige signalreiche Areale, Darstellung nach 1 und 10 Minuten (1 min, 10 min). Nach etwa 50 Minuten (50 min) ist nahezu der gesamte Farbstoff DY635 aus der Leber ausgeschieden. Ähnliche Ergebnisse zeigt DY635. **B:** Insgesamt zeigt sich ein ähnliches Bild wie bei den früheren Untersuchungen mit dem reinen Farbstoff DY635. **A:** zeigt die Abklingrate von DY635 in der Leber. Die geänderte Abklingrate der DY635-Intensität in der Leber von DY635 und DY635-PLGA-NP zeigt, dass der Farbstoff DY635 auch intrazellulär noch an das Polymer PLGA gebunden ist und erst nach Hydrolyse des PLGA frei wird und ausgeschieden wird. **D-G:** zeigt die Organspezifitäten, die mittels verschiedener Organschnitte überprüft wurde. In der Leber **(D)** ist nach Injektion von DY635-PLGA-NP (grün) eine starke Akkumulation festzustellen. In der Milz **(E),** im Herz **(F)** und in den Nieren **(G)** sind dagegen kaum (Milz, Herz) bis keine (Niere) Nanopartikel sichtbar.

### Beispiel 6: Sekretionsroute des Nanopartikels DY635-PLGA

Anhand dieses Experiments wurde die Plasmaverschwinderate und die Gallesekretion der DY635-PLGA-Nanopartikel bzw. des Polymethinfarbstoffs DY635 untersucht. Hierfür werden männliche Ratten (Stamm: RccHan:WIST) instrumentiert (Katheter in V. jugularis, A. carotis, Ductus choledochus). Anschließend wird die zu testende Substanz über den venösen Katheter injiziert. Anschließend werden in kurzen Zeitintervallen Blut aus dem arteriellen Katheter und Galle aus dem Katheter im Ductus choledochus entnommen. Das Blut wird hinterher weiter zu Plasma aufgearbeitet. Die Menge des Farbstoffs DY635 wird anschließend durch Fluorimetrie über eine Eichkurve gemessen. DY635-PLGA-NP waren nach 4 Minuten maximal im arteriellen Blut zu finden und wurden bis 20 Minuten nach DY635-PLGA-NP-Injektion, also innerhalb von 15 Minuten (min), fast vollständig in Organe aufgenommen. Leicht verzögert, da, wie bereits beschrieben, DY635 zuerst aus den Nanopartikeln freigesetzt werden muss, wird DY635 in die Galle sezerniert (Figur 6, Abbildung **A**). Die (rechnerische) Recovery von DY635 in der Galle von 95% zeigt ebenfalls die hohe Spezifität der DY635-PLGA-NP für Hepatozyten (Figur 6, Abbildung **B**).

### Beispiel 7: Einschluss von pharmazeutischen Wirkstoffen in Nanopartikel

Nach der Funktionalisierung der Polymere oder Lipide mit der Targeingeinheit (Beispiel 1) werden durch einfache (A) und doppelte (B) Emulsion Nanopartikel hergestellt.

### (A) Nanopartikel aus einfacher Emulsion

Sollten hydrophile Substanzen eingeschlossen werden, wurde die Singelemulsion Technik verwendet. Hier ist der Wirkstoff durch hydrophobe Interaktionen in einen hydrophoben Polymerkern eingeschlossen. Der Wirkstoff wurde dabei mit dem Polymer in einem geeigneten organischen Lösungsmittel gelöst. Geeignet ist ein organisches Lösungsmittel, wenn es sowohl das Polymer als auch dem Wirkstoff gegenüber neutral ist, d.h. diesen chemisch nicht verändert und keinen Einfluss auf deren Stabilität hat. Vorliegend wurde Ethylacetat verwendet. Das Gemisch wurde mit einer hydrophilen Lösung überschichtet. Zur Stabilisierung der Nanopartikel und zur Erhöhung der Ausbeute kann wie bei Doppelemulsions Nanopartikel der hydrophilen Lösung ein Surfactant beigemengt werden (vgl. Doppelemulsion Nanopartikel). Die zwei Phasen wurden durch hochenergetischen Ultraschall, welcher koaxial zu einer senkrecht in die Probe eingetauchten Elektrode ausgestrahlt wird, gemischt. Dadurch entstanden Nanopartikel.

### (B) Doppelemulsion Nanopartikel

Zur Herstellung wurden die hydrophoben Polymere in hoher Konzentration in einem geeigneten Lösungsmittels gelöst. Geeignet ist ein organisches Lösungsmittel, wenn es sowohl das Polymer als auch dem Wirkstoff gegenüber neutral ist, d.h. diesen chemisch nicht verändert und keinen Einfluss auf deren Stabilität hat. Vorliegend wurde Ethylacetat verwendet. Die Konzentration der Polymere ist abhängig von der Größe, Hydrophilie, Löslichkeit und Stabilität des Polymers. Geeignete Konzentrationen liegen hier zwischen 2 und 50 mg/mL. Der Wirkstoff wurde in geeigneter Konzentration in Reinstwasser gelöst. Eine geeignete Wirkstoffkonzentration hängt von den chemischen Eigenschaften des Wirkostoffs und der Kapazität der Nanopartikel ab. Im Anschluss wurde das in dem organischem Lösungsmittel gelöste Hüllpolymer mit dem in wässriger Lösung gelösten Wirkstoff überschichtet. Das Polymer und organische Lösungsmittel musste dabei im mindestens 10-Fachen Überschuss in der Probe vorhanden sein. Durch das Einstrahlen von hochenergetischem Ultraschall koaxial zu einer senkrecht in die Probe getauchte Elektrode, entstanden nach außen hin hydrophobe Nanopartikel. Der Wirkstoff wurde dadurch durch Interaktion mit hydrophilen Gruppen des Nanopartikels im inneren in einen hydrophilen Kern eingeschlossen. Im zweiten Schritt wurde ein geeignetes Surfactant in geeigneter Konzentration in Reinstwasser gelöst. Eine Surfactant-Konzentration ist adäquat, wenn Sie ausreichend Nanopartikel herzustellen. Die Konzentration hängt von den Umgebungsbedingungen ab und muss experimentell ermittelt werden. Sie liegt in der Regel zwischen 0.01 und 5% (w/v). Dann wurde so viel Surfactant zu der Probe gegeben, dass die Konzentration an Polymer nur noch mindestens 1/10 der Ausgangsmenge betrug. Wieder bildeten sich zwei Phasen, die durch hochfrequenten Ultraschall, der koaxial zu einer senkrecht in die Probe getauchten Elektrode ausgestrahlt gemischt wurde. Durch Beimengung der Oberflächen-aktiven Substanzen (Surfactant), z.B. Tenside, vorliegend Polyvinylakohol, wurde die Bildung von wasserlöslichen nanoskaligen Partikeln gewährleistet.

Zur Veranschaulichung wird ein Ansatz beschrieben in dem hydrophile, mit Polyethylenimin (PEI) komplexierte, *small interferin RNA* (siRNA) in PLGA-Nanopartikel eingeschlossen wurden. Das PLGA wurde vorab mit DY-635 modifiziert, sodass jede 200ste Kette ein Farbstoffmolekül trägt:
(1) 2,4 µL PEI (1 mg/ml) wurden mit 2 µl siRNA (1 µg/µl) gemischt und in mit 45,6 µl Reinstwasser gemischt. Das Gemisch wird im Folgenden als Polyplexe bezeichnet, da die anionische siRNA und das kationische PEI miteinander interagiert und ein PEI die siRNA in einem engmaschigen Netzwerk bindet und stabilisiert.
(2) 325 mg DY-635 konjugiertes PLGA wurden in insgesamt 12,35 ml Ethylacetat gelöst.
(3) 90 µl Polymerlösung aus (2) wurden mit 50 µl Polyplexen aus (1) mit hochfrequentem Ultraschall (wie oben beschrieben eingestrahlt) gemischt.
(4) 1 mL PVA 0,3 wt% in Reinstwasser wurde zu dem Gemisch hinzugefügt und Ultraschall eingestrahlt.
(5) Die Entstandenen Nanopartikel wurden gereinigt und gefriergetrocknet.

### Aufreinigung (für (A) und (B)

Die so hergestellten Nanopartikel hatten einen Durchmesser, der abhängig von Form und Material der Gefäßen, Stärke des Ultraschalls und Stoffkonzentration war und besaßen eine Größe von 120 bis 220 nm. Unter sterilen Bedingungen wurde nach Herstellung der Nanopartikel das Lösungsmittel entfernt. Um das überschüssige Tensid zu entfernen, wurden die Nanopartikel mehrmals (mindestens zwei Mal) durch Zentrifugieren, abnehmen des Überstands und Resuspendieren der Nanopartikel in sterilem Reinstwasser gewaschen. Abschießend wurden die Partikel lyophilisiert und deren Masse bestimmt.

### Beispiel 8: Einschluss von pharmazeutischen Wirkstoffen in Liposomen

Nach der Funktionalisierung der Polymere oder Lipide mit der Targeingeinheit (Beispiel 1) wurden Liposomen wie folgt hergestellt.
1. Herstellung einer 50 mM Lipidlösung aus z.B 1:1 DOPC:DSPC (1,2-Dioleolyl-sn-glycero-3-phosphocholine:1,2-Distearoi-sn-glycero-3-phospholine) + 30% cholesterol + 5% N-dod-DOPE in Chloroform/Methanol (2:1 vol/vol). Das DOPC kann vor Verwendung mit einem Polymethinfarbstoff modifiziert werden.
2. Verdampfen des Chloroform/Methanol-Lösungsmittel (ca. 30 min, 90 rpm) in einem Rotationsverdampfer.
3. Die Lipide wurden anschließend in 1 ml 7:3 vol/vol gemischtem DMSO:EtOH gelöst.
4. Anschließend wurd als Wirkstoff das hydrophile Dextran in einem geeigneten Puffer, und zwar PBS (*Photphate Buffered Solution*) zu einer Konzentration von 1 mg/ml gelöst.
5. 0,3 ml der Lipidlösung werden dann tropfenweise zu der Dextranlösung geben. Die Dextranlösung wird während des dazu-tropfen bei 750 rpm auf einem Magnetrührer in Bewegung gehalten.
6. Die Liposomen werden anschließend in einem Mini-Extruder getrennt
7. Anschließend wird die Liposomenlösung in 1 ml Gefäßen aliquotiert und 10 Mal abwechselnd in flüssigem Stickstoff gefroren und in warmen Wasser wieder aufgetaut.
8. Anschließend werden die Liposomen nochmals 10 Mal im extruder getrennt.
9. Dann warden die Liposomen in einer vorbereiteten Dialyse-Kassette (MWCO = 20 kDa) gegen PBS für 16 Stunden dialysiert
10. Anschließend können die Liposomen gefriergetrocknet, gelagert oder verwendet werden.

### Beispiel 9: Beeinflussung der Cholesterolbiosynthese durch den organspezifischen Transport einer siRNA gegen die HMG-CoA Reduktase (HMGCR) in DY-635 modifitierten nanostrukturiertes Trägersystemen

Männlichen FVB/NRj Mäusen (10 Wochen alt) wurden zwei Mal im Abstand von 24 Stunden mit dem DY-635 modifizierten nanostrukturierten Trägersystem durch i.v. Injektion behandelt. Dabei wurden 6,5 µg des nanostrukturierten Trägersystems pro g Körpergewicht injiziert. Die Herstellung des Trägersystems erfolgte wie in Beispiel 7 (B) beschrieben, wobei für die Herstellung 3 µg siRNA gegen die HMGCR oder 3 µg *scrambeled* siRNA (siRNA ohne Wirkung) mit 108 µg PEI in 3 mg DY-635 modifiziertem PLGA eingeschlossen wurde. 16 Stunden nach der zweiten Injektion wurden die Tiere schmerzlos euthanasiert und Blut, sowie Organe zur Analyse entnommen. Das Blut wurde in Lithium-Heparin Monovetten abgenommen und zu Plasma aufbereitet. Zur Wirksamkeit der Therapie wurde das Gesamtcholesterin im Plasma bestimmt, sowie für die Spezifität die Genexpressionsänderung in verschiedenen Organen in der qPCR bestimmt. Diese Werte werden mit dem Cholesterin und dem HMGCR Expressionsniveau gesunder FVB/NRj Mäuse (10 Wochen alt) und Kontrollgruppen verglichen. Die Kontrollgruppen setzen sich folgendermaßen zusammen:
- Behandlung mit einem DY-635 modifizierten und damit hepatozytenspezifischen nanostrukturierten Trägersystem und einer unwirksamen *scrambled* siRNA
- Behandlung mit einem nanostrukturierten Trägersystem, welches keine DY-635 Modifikation enthält und sich sonst nicht von dem therapeutischen Konstrukt unterschiedet
- Tiere die lediglich die 5%ige Glukoselösung erhielten.

### Beispiel 10 Nachweis der Interaktion von DY-635 mit hepatozytären Transportern

HEK-293T Zellen wurden mit humanen gewebsspezifischen hepatozytären Transportern transfiziert. Anschließend wurden für **Figur 10** **A** die Aufnahme des Polymethinfarbsoffs DY-635 als Targetingeinheit in diese gewebsspezifischen Transporter untersucht. Hierfür wurden die Zellen auf 96-Well-Platten ausgesät, 24 Stunden unter Standardbedingungen inkubiert, nach einem Mediumwechsel 5 Minuten mit DY-635 (Endkonzentration im Well: 10 µmol/l) inkubiert. Anschließend wurden die Zellen lysiert und die Lysate in der Fluorimetrie vermessen. Durch eine DY-635 Standardkurve konnte die aufgenommenen DY-635 Menge quantifiziert werden. Zur Kontrolle wurden die jeweiligen Transporter spezifisch inhibiert (Inhibitoren und verwendete Endkonzentrationen sind in nachstehender **Tabelle 3** beschrieben). In diesem Versuch zeigte sich, dass DY-635 ein Substrat für NTCP ist. Die Aufnahme durch OCT1 kann als vernachlässigbar bewertet werden. In **Figur 10** **B** wurde untersucht, ob DY-635 als Inhibitor an die basolateralen hepatozytären Transporter bindet. Hierfür wurden die mit den gewebsspezifischen Transportern transfizierten HEK-293T Zellen wie beschrieben ausgesät und inkubiert. Nach 24 Stunden wurden die Zellen entweder mit einem radioaktiv markierten, Transporter-spezifischem Substrat, oder dem radioaktivenspezifischen Substrat mit einem spezifischen Inhibitor oder mit DY-635 (10 µmol/l Endkonzentration) für 5 Minuten inkubiert (die verwendeten Substrate und deren Konzentration sind in nachfolgender **Tabelle 3** beschrieben). Die Zellen wurden anschließend gewaschen und im Well lysiert. Für die Quantifizierung der Aufnahme wurde die radioaktive Strahlung der Substrate verwendet. Hier zeigte sich, dass DY-635 ein starker Inhibitor für OATP1B1 und OATP1B3 ist. Auch OAT2 und OCT1 werden durch DY-635 inhibiert. Dies verdeutlicht die starke Interaktion von DY-635 mit den gewebsspezifischen hepatozytären Transportern. Es kann geschlussfolgert werden, dass durch die Exposition des DY-635 an der Oberfläche eines nanostrukturierten Transportsystems es zur Immobilisierung dieses an der Zelloberfläche der Hepatozyten kommt, was zur konsekutiven Endozytose des Nanopartikels führt.

**Tabelle 3**

| **Humaner hepatozytärer Transporter** | **Radioaktiv markiertes Transporterspezifisches Substrat/ Konzentration** | **Transporter-spezifischer Inhibitor/ Konzentration** |
|---|---|---|
| OATP1B1 | [³H]Estradiol/ 30 nM | Rifampicin/ 5 µM |
| OATP1B3 | [³H]Sulfobromophthalein/ 50 nM | Rifampicin/ 5 µM |
| OAT2 | [³H]cGMP/ 10 nM | Indomethacine/100 µM |
| NTCP | [³H]Estradiol/ 30 nM | Cyclosporin A/ 50 µM |
| NaDC3 | [¹⁴C]Succinat/10 µM | Succinat/100 µM |
| OCT1 | [³H] 1-Methyl-4-phenylpyridinium | Decynium22/ 50 µM |

## Patentansprüche

1. Nanostrukturiertes Trägersystem umfassend mindestens ein Polymer und/oder mindestens ein Lipid und mindestens einen Polymethinfarbstoff zur Verwendung für die Behandlung von Erkrankungen der Leber und/oder Niere, wobei der mindestens eine Polymethinfarbstoff als Targetingeinheit den zielgerichteten Transport des nanostrukturieren Trägersystems in ein Zielgewebe bewirkt, und wobei der mindestens eine Polymethinfarbstoff ein symmetrisches oder unsymmetrisches Polymethin der allgemeinen Struktur I, II, III oder IV ist: 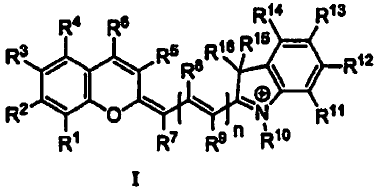 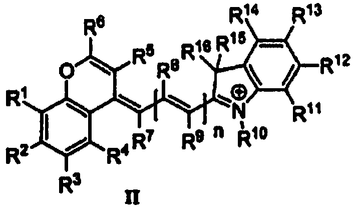 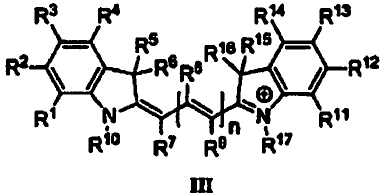 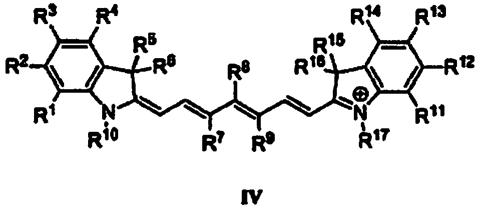 wobei
a. n für die Zahlenwerte 0, 1, 2, 3, 4, 5, 6, 7, 8, 9, oder 10 steht,
b. R¹- R¹⁷ gleich oder unterschiedlich sind und Wasserstoff, ein oder mehrere Alkyl-, tert-Alkyl, Cycloalkyl oder Aryl-, Carboxyaryl-, Dicarboxyaryl-, Heteroaryl- oder heterocycloaliphatische Reste, Alkyloxy-, Alkylmercapto, Aryloxy-, Arylmercapto-, Heteroaryloxy-, Heteroarylmercapto-, eine Hydroxy-, Nitro- oder Cyanogruppe, eine alkylsubsituierte oder cyclische Aminfunktion sein können und/oder zwei ortho-ständige Reste, z.B. R³ und R⁴, R¹³ und R¹⁴ und/oder R¹ und R² und R¹¹ und R¹² und/oder R⁷ und R⁹ zusammen einen weiteren aromatischen, hetero-aromatischen, aliphatischen oder heteroaliphatischen Ring bilden können,
c. mindestens einer der Substituenten R¹- R¹⁷ trägt einen solubilisierenden bzw. ionisierbaren oder ionisierten Substituenten, wie SO₃⁻, (-SOsH), PO₃²⁻, COOH, OH oder NR₃⁺, Cyclodextrine oder Zucker, der die hydrophilen Eigenschaften dieser Polymethinfarbstoffe bestimmt, wobei dieser Substituent auch über eine Spacergruppe an dem Polymethinfarbstoff angebunden sein kann, und
d. mindestens einer der Substituenten R¹- R¹⁷ trägt eine reaktive Gruppe (Linker), wie Isocyanate, Isothiocyanate, Hydrazine, Amine, Mono- und Dichlor- oder Mono- und Dibromtriazine, Aziridine, Epoxide, Sulfonylhalogenide, Säurehalogenide, Carbonsäureanhydride, N-Hydroxysuccinimidester, Imido-ester, Carbonsäuren, Glyoxal, Aldehyd, Maleimid oder lodacetamid und Phosphoramidit Derivate oder Azide, Alkine oder Olefine, wobei dieser Substituent auch über eine Spacergruppe an dem Polymethinfabstoff angebunden sein kann,
e. die aromatische, heteroaromatische, aliphatische oder heteroaliphatische Spacergruppe aus Strukturelementen, wie -[(CH₂)ₐ-Y-(CH₂)_{b}]_{c}- oder-[(C₆H₄)ₐ- Y-(C₆H₄)_{b}]- besteht, wobei Y gleich oder unterschiedlich ist und CR₂-, O-, S-, SO₂, SO₂NH-, NR-, 000- oder CONR Funktionen beinhalten, wobei sie an einem der Substituenten R¹- R¹⁷ ist und a und b gleich oder unterschiedlich sind mit Zahlenwerte von 0-18 und mit Zahlenwerte für c von 0-18,
f. die Substituenten R⁸ und R⁹ bei entsprechenden n= 2, 3, 4 oder 5 ebenfalls 2-,3-,4- oder 5-fach vorhanden sein können und diese gleich oder unterschiedlich sein können, und wobei
das nanostrukturierte Trägersystem weiterhin mindestens einen pharmazeutischen Wirkstoff umfasst.

2. Nanostrukturiertes Trägersystem zur Verwendung nach Anspruch 1, wobei der mindestens eine Polymethinfarbstoff über mindestens einen gewebsspezifischen Transporter die Aufnahme des nanostrukturieren Trägersystems in die Zellen des Zielgewebes bewirkt.

3. Nanostrukturieres Trägersystem zur Verwendung nach einem der vorangehenden Ansprüche, wobei der mindestens eine Polymethinfarbstoff ausgewählt ist aus der Gruppe bestehend aus DY635, DY-680, DY-780, DY-880, DY-735, DY-835, DY-830, DY-730, DY-750, DY-850, DY-778, DY-878, DY-704, DY-804, DY-754, DY-854, DY-700, DY-800, ICG und DY-IRDYE 800CW.

4. Nanostrukturieres Trägersystem zur Verwendung nach einem der vorangehenden Ansprüche, wobei das mindestens eine Polymer ausgewählt ist aus der Gruppe bestehend aus Polyestern, Poly(meth)acrylaten, Polystyrol Derivaten, Polyamiden, Polyurethanen, Polyacrylnitrilen, Polytetrafluorethylenen, Siliconen, Polyethylenglycolen, Polyethylenoxiden und Polyoxazolinen und deren Copolymere, bevorzugt in verschiedenster Zusammensetzung, wie Statistisch, Gradient, Alternierend, Block, Pfropf oder Stern Copolymere, oder das mindestens eine Lipid ausgewählt ist aus der Gruppe bestehend aus gesättigten und ungesättigten Fettsäuren, bevorzugt Cholesterin, Palmethylsäure, Phospolipide, Sphingolipide und Glycolipide.

5. Nanostrukturieres Trägersystem zur Verwendung nach einem der vorangehenden Ansprüche, wobei der mindestens eine gewebsspezifische Transporter ausgewählt ist aus der Gruppe bestehend aus OATP1B1, OATP-C, OATP2, LST-1, OATP1B3, OATP8, OATP2B1, OATP1A2, NaDC3, SDCT2, NTCP, OCT1, OCT3, OAT2, OAT1, OAT3, PGT, OCT2, OAT1, OATP4A1, OATP4C1.

6. Nanostrukturieres Trägersystem zur Verwendung nach einem der vorangehenden Ansprüche, wobei der mindestens eine pharmazeutische Wirkstoff ausgewählt ist aus der Gruppe bestehend aus niedermolekularen Substanzen, insbesondere Induktoren oder Kontrastmittel, und höhermolekularen Substanzen, insbesondere Nukleinsäuren und Proteinen, bevorzugt ausgewählt aus der Gruppe bestehend aus Glucokortikoiden, Zytostatika, Antimetaboliten, Interkalantien, Antikörper, Interferone, Phospho-Inositol-3-Kinase-Inhibitoren, Coxibe, JNK-Inhibitoren.

7. Nanostrukturiertes Trägersystem zur Verwendung nach einem der vorangehenden Ansprüche, wobei die Akkumulation des nanostrukturieren Trägersystems und/oder seiner Bestandteile in einem Zielgewebe mittels Fluoreszenzeigenschaften des mindestens einen Polymethinfarbstoffs detektierbar ist.

8. Nanostrukturieres Trägersystem zur Verwendung nach einem der Ansprüche 1 bis 7, wobei die Erkrankung ausgewählt ist aus der Gruppe, bestehend aus Infektionserkrankungen mit Schädigung der Leber und/oder Niere, Leberversagen, Leberzirrhose, Stoffwechselerkrankungen der Leber, exkretorische Dysfunktionen der Leber, Lebertumore, primäre Lebertumore, Nierentumore, primäre Nierentumore, Nephritiden, chronisches und akutes Nierenversagen sowie Erkrankungen die eine konsekutive Schädigung der Leber und/oder der Niere verursachen.

## Claims

1. Nanostructurered delivery system, comprising at least one polymer, and/or at least one lipid and at least one polymethine dye for use in the treatment of diseases of the liver and/or the kidneys, wherein the at least polymethine dye, as a targeting unit, causes the transport of the nanostructured delivery system into the target tissue, and wherein the at least one polymethine dye is a symmetrical or asymmetrical polymethine of the general structure I, II, III or IV: 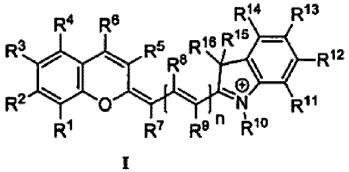 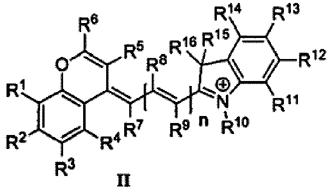 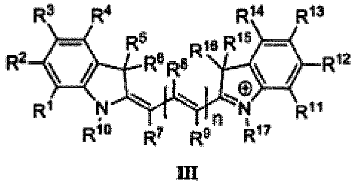 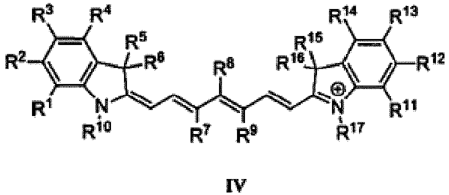 wherein
a. n stands for the numerical values 0, 1, 2, 3, 4, 5, 6, 7, 8, 9 or 10,
b. R¹-R¹⁷ may be the same or different and maybe hydrogen, one or more alkyl, tert-alkyl, cycloalkyl or aryl-, carboxyaryl-, dicarboxyaryl-, heteroaryl- or heterocycloaliphatic radicals, alkyloxy-, alkylmercapto, heteroaryloxy-, heteroarylmercapto-, hydroxyl-, nitro- or cyano- group, an alkyl-substituted or cyclic amine function and/or two ortho-position radicals, e.g., R³ and R⁴, R¹³ and R¹⁴ and/or R¹ and R² and R¹¹ and R¹² and/or R⁷ and R⁹ together may form an additional aromatic, heteroaromatic, aliphatic or heteroaliphatic ring,
c. at least one of the R¹ -R¹⁷ substituents has a solubilizing and/or ionizable or ionized substituent such as SO₃⁻ , (- SO₃H), PO₃²⁻, COOH, OH or NR₃⁺, cyclodextrins or sugar, which determines the hydrophilic properties of these polymethine dyes, wherein this substituent may be bound to the polymethine dye also by a spacer group, and
d. at least one of the R¹-R¹⁷ substituents has a reactive group (linker) such as isocyanates, isothiocyanates, hydrazines, amines, mono- and dichloro- or mono- and dibromotriazines, aziridines, epoxides, sulfonyl halides, acid halides, carboxylic anhydrides, N-hydroxysuccinimide esters, imido esters, carboxylic acids, glyoxal, aldehyde, maleimide or iodacetamide and phosphoramidite derivatives or azides, alkynes or olefins, wherein this substituent may be bound to the polymethine dye also by a spacer group,
e. the aromatic, heteroaromatic, aliphatic or heteroaliphatic spacer group consists of structural elements such as [(CH₂)ₐ) - Y- (CH₂)_{b}]_{c} or [(C₆H₄)ₐ) - Y- (C₆H₄)_{b}], where Y may be the same or different and comprises CR₂-, O-, S-, SO₂, SO₂NH-, NR-, COO- or CONR functions, wherein it is bound to one of the R¹-R¹⁷ substituents, and a and b may be the same or different and have numerical values of 0-18 and numerical values for c of 0-18,
f. the R⁸ and R⁹ substituents with corresponding n=2, 3, 4 or 5, may also be present 2x, 3x, 4x or 5x, and wherein these may be the same or different, and wherein
the nanostructured delivery system comprises at least pharmaceutically active ingredient.

2. Nanostructured delivery system for the use according to claim 1, wherein the at least one polymethine dye causes the uptake of the nanostructured delivery system into the cells of the target tissue via at least one tissue-specific transporter.

3. The nanostructured carrier system for the use according to any one of the preceding claims, wherein the at least one polymethine dye is selected from the group consisting of DY-635, DY-680, DY-780, DY-880, DY-735, DY-835, DY-830, DY-730, DY-750, DY-850, DY-778, DY-878, DY-704, DY-804, DY-754, DY-854, DY-700, DY-800, ICG and DY-IRDYE 800CW.

4. The nanostructured delivery system for the use according to any one of the preceding claims, wherein the at least one polymer is selected from the group consisting of polyesters, poly(meth)acrylates, polystyrene derivatives, polyamides, polyurethanes, polyacrylonitriles, polytetrafluoroethylenes, silicones, polyethylene glycols, polyethylene oxides and polyoxazolines and copolymers thereof, preferably in various compositions, such as random, gradient, alternating, block, graft or stem copolymers, or wherein that at least one lipid is selected from the group consisting of saturated and unsaturated fatty acids, preferably cholesterol, palmitic acid, phospholipids, sphingolipids and glycolipids.

5. The nanostructured delivery system for the use according to any one of the preceding claims, wherein the at least one tissue-specific transporter is selected from the group consisting of OATP1B1, OATP-C, OATP2, LST-1, OATP1B3, OATP8, OATP2B1, OATP1A2, NaDC3, SDCT2, NTCP, OCT1, OCT3, OAT2, OAT1, OAT3, PGT, OCT2, OAT1, OATP4A1, OATP4C1.

6. Nanostructured delivery system for the use according to any one of the preceding claims, wherein the at least one pharmaceutically active ingredient is selected from the group consisting of low-molecular substances, in particular inducers or contrast agents, and higher molecular substances, in particular nucleic acids and proteins, preferably selected from the group consisting of glucocorticoids, cytostatics, antimetabolites, intercalants, antibodies, interferons, phosphoinositol 1-3 kinase inhibitors, coxibes, JNK inhibitors.

7. The nanostructured delivery system for the use according to any of the preceding claims, wherein the accumulation of the nanostructured delivery system and/or its components in a target tissue is detectable by means of the fluorescent properties of the at least one polymethine dye.

8. The nanostructured delivery system for the use according to any one of claims 1 to 7, wherein the disease is selected from the group consisting of infectious diseases with damage to the liver and/or kidney, liver failure, liver cirrhosis, metabolic diseases of the liver, excretory dysfunctions of the liver, liver tumors, primary liver tumors, kidney tumors, primary kidney tumors, nephritis, chronic and acute kidney failure and diseases, which cause a consecutive damage to the liver and/or kidney.

## Revendications

1. Système porteur nanostructuré comprenant au moins un polymère et/ou au moins un lipide et au moins un colorant polyméthinique pour son utilisation pour le traitement de maladies du foie et/ou des reins, dans lequel l'au moins un colorant polyméthinique provoque en tant qu'une unité de ciblage le transport ciblé du système porteur nanostructuré dans un tissu ciblé, et dans lequel l'au moins un colorant polyméthinique est une polyméthine symétrique ou asymétrique de la structure générale I, II, III ou IV : 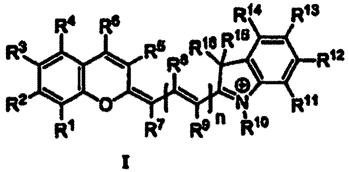 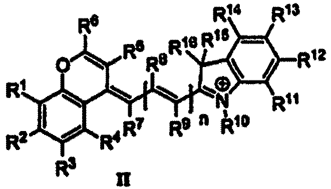 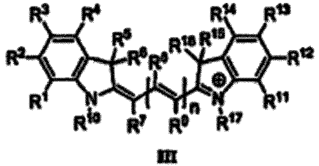 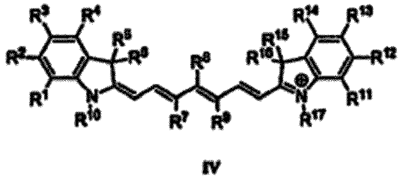 dans lequel
a. n représente les valeurs numériques 0, 1, 2, 3, 4, 5, 6, 7, 8, 9 ou 10,
b. R¹-R¹⁷ sont identiques ou différents et peuvent former de l'hydrogène, un ou plusieurs radicaux alkyle, tert-alkyle, cycloalkyle ou aryle, carboxyaryle, dicarboxyaryle, hétéroaryle ou hétérocycloaliphatique, un groupe alkyloxy, alkylmercapto, aryloxy, arylmercapto, hétéroaryloxy, hétéroarylmercapto, un groupe hydroxy, nitro ou cyano, une fonction amine alkylasubstituée ou cyclique et/ou peuvent former deux radicaux en position β, par exemple R³ et R⁴, R¹³ et R¹⁴ et/ou R¹ et R² et R¹¹ et R¹² et/ou R⁷ et R⁹, peuvent former conjointement un autre cycle aromatique, hétéroaromatique, aliphatique ou hétéroaliphatique,
c. au moins un des substituants R¹-R¹⁷ supporte un substituant de solubilisation ou d'isonisation ou ionisé, tel que SO₃⁻ (-SO₃H), PO₃²⁻, COOH, OH ou NR₃⁺, de la cyclodextrine ou du sucre, qui définit les propriétés hydrophiles desdits colorants polyméthiniques, dans lequel ledit substituant peut être attaché au colorant polyméthinique également par l'intermédiaire d'un groupe écarteur, et
d. au moins un des substituants R¹-R¹⁷ supporte un groupe réactif (liant) tel que des isocyanates, des isothiocyanates, des hydrazines, des amines, des mono-, dichloro ou mono- et dibromotriazines, des aziridines, des époxydes, des halogénures de sulfonyle, des halogénures d'acide, des anhydrides d'acide carboxylique, de l'ester de N-hydroxysuccinimide, de l'ester imido, des acides carboxyliques, du glyoxal, de l'aldéhyde, des dérivés de maléimide ou d'iodoacétamide et de phosphoramidite ou des azides, des alkynes ou des oléfines, dans lequel ledit substituant peut être attaché au colorant polyméthynique également par l'intermédiaire d'un groupe écarteur,
e. le groupe écarteur aromatique, hétéroaromatique, aliphatique ou héréroaliphatique est constitué d'éléments structurels, tels que -[(CH₂)ₐ-Y-(CH₂)_{b}]_{c}- ou -[(C₆H₄)ₐ-Y-(C₆H₄)_{b}]-, dans lequel Y est identique ou différent et CR₂-, O-, S-, SO₂, SO₂NH-, NR-, 000- ou CONFR contiennent des fonctions, dans lequel il est au niveau d'un des substituants R¹-R¹⁷ et a et b sont identiques ou différents avec des valeurs numériques de 0 à 18 et avec des valeurs numériques pour c de 0 à 18,
f. les substituants R⁸ et R⁹ peuvent être présents pour n correspondants - 2, 3, 4 ou 5 également en une quantité x2, x3, x4 ou x5 et ceux-ci peuvent être identiques ou différents, et dans lequel
le système porteur nanostructuré comprend par ailleurs au moins un principe actif pharmaceutique.

2. Système porteur nanostructuré pour son utilisation selon la revendication 1, dans lequel l'au moins un colorant polyméthinique entraîne par l'intermédiaire d'au moins un transporteur spécifique au tissu l'absorption du système porteur nanostructuré dans les cellules du tissu ciblé.

3. Système porteur nanostructuré pour son utilisation selon l'une quelconque des revendications précédentes, dans lequel l'au moins un colorant polyméthinique est choisi parmi le groupe constitué de DY635, DY-680, DY-780, DY-880, DY-735, DY-835, DY-830, DY-730, DY-750, DY-850, DY-778, DY-878, DY-704, DY-804, DY-754, DY-854, DY-700, DY-800, ICG et DY-IRDYE 800CW.

4. Système porteur nanostructuré pour son utilisation selon l'une quelconque des revendications précédentes, dans lequel l'au moins un polymère est choisi parmi le groupe constitué de polyesters, de poly(méth)acrylates, de dérivés de polystyrène, de polyamides, de polyuréthanes, de polyacrylonitriles, de polytétrafluoroéthylènes, de silicones, de polyéthylèneglycols, de polyoxydes d'éthylène et de polyoxazolines et de leurs copolymères, de manière préférée en une composition la plus variée, tels que des copolymères statiques, à gradient, à alternance, en bloc, greffés ou en étoile, ou l'au moins un lipide est choisi parmi le groupe constitué d'acides gras saturés et insaturés, de manière préférée de cholestérine, d'acide de palméthyle, de phospholipides, de sphingolipides et de glycolipides.

5. Système porteur nanostructuré pour son utilisation selon l'une quelconque des revendications précédentes, dans lequel l'au moins un transporteur spécifique au tissu est choisi parmi le groupe constitué de OATP1B1, OATP-C, OATP2, LST-1, OATP1B3, OATP8, OATP2B1, OATP1A2, NaDC3, SDCT2, NTCP, OCT1, OCT3, OAT2, OAT1, OAT3, PGT, OCT2, OAT1, OATP4A1, OATP4C1.

6. Système porteur nanostructuré pour son utilisation selon l'une quelconque des revendications précédentes, dans lequel l'au moins un principe actif pharmaceutique est choisi parmi le groupe constitué de substances à faible poids moléculaire, en particulier d'inducteurs et d'agents contrastants et de substances à poids moléculaire élevé, en particulier d'acides nucléiques et de protéines de manière préférée choisi parmi le groupe constitué de glucocorticoïdes, de cytostatiques, d'antimétabolites, d'intercalants, d'anticorps, d'interférons, d'inhibiteurs phosphoinositol-3-kinase, de coxibes, d'inhibiteurs JNK.

7. Système porteur nanostructuré pour son utilisation selon l'une quelconque des revendications précédentes, dans lequel l'accumulation du système porteur nanostructuré et/ou de ses constituants dans un tissu ciblé peut être détectée au moyen de propriétés de fluorescence de l'au moins un colorant polyméthinique.

8. Système porteur nanostructuré pour son utilisation selon l'une quelconque des revendications 1 à 7, dans lequel la maladie est choisie parmi le groupe constitué de maladies infectieuses endommageant le foie et/ou les reins, de l'insuffisance hépatique, de la cirrhose, de maladies métaboliques du foie, de dysfonctionnement excrétoire du foie, de tumeurs hépatiques, de tumeurs hépatiques primaires, de tumeurs rénales, de tumeurs rénales primaires, de néphrites, d'insuffisance rénale chronique et aiguë ainsi que de maladies, qui provoquent des effets secondaires sur le foie et/ou les reins.
